# EUROPEAN PATENT APPLICATION

(11) **EP 4 527 465 A2**
(43) Date of publication of application: **26.03.2025**
(21) Application number: 25152478.1
(22) Date of filing: 17.10.2022
(51) Int. Cl.: A61P 35/00

(54) **ANTI-GLYPICAN 3 ANTIBODIES**

(30) Priority: 15.10.2021 WO PCT/CN2021/124182
(62) Divisional of application: 22880441.5
(71) Applicant: Concept to Medicine Biotech Co., Ltd., Shanghai 201203 (CN)
(72) Inventor: YANG, Liu, Shanghai, 201203 (CN); SHEN, Hao, Shanghai, 201203 (CN); CUI, Feifei, Shanghai, 201203 (CN); FANG, Lei, Shanghai, 201203 (CN)
(74) Representative: Gleiss Große Schrell und Partner mbB

(57) **Abstract**

This disclosure provides anti-GPC3 antibodies, including murine antibodies, humanized antibodies, and those with further optimized CDR sequences. The newly disclosed antibodies exhibited higher cell-based binding efficiency than the leading anti-GPC3 antibody candidate under clinical development.

## Description

### BACKGROUD

Glypican 3 (GPC3) is a member of the glypican-related integral membrane heparan sulfate proteoglycans (GRIPS) family that are present on cell surfaces. The protein core of GPC3 consists of two subunits, where the N-terminal subunit has a size of ~40 kDa and the C-terminal subunit is ~30 kDa. Six glypicans (GPC1-6) have been identified in mammals. GPC3 plays an important role in modulating the cell proliferation, differentiation, adhesion and migration. GPC3 interacts with both Wnt and frizzled (FZD) to form a complex and triggers downstream signaling. The core protein of GPC3 may serve as a co-receptor or a receiver for Wnt.

Glypican 3 immunostaining can be for differentiating hepatocellular carcinoma (HCC) and dysplastic changes in cirrhotic livers. GPC3 protein expression is found in HCC, not in normal liver and cholangiocarcinoma. GPC3 is also expressed to a lesser degree in melanoma, ovarian clear-cell carcinomas, yolk sac tumors, neuroblastoma, hepatoblastoma, Wilms' tumor cells, and other tumors.

GPC3 is a promising therapeutic target for treating cancers such as liver cancer. Several therapeutic anti-GPC3 antibodies have been developed, including GC33 and YP7. Some of these antibodies inhibit Wnt signaling in liver cancer cells. Also, chimeric antigen receptor (CAR) T cell immunotherapies are being developed at various stages for treating cancer. In mice with xenograft or orthoptic liver tumors, CAR-T cells can eliminate GPC3-positive cancer cells, by inducing perforin- and granzyme-mediated cell death and reducing Wnt signaling in tumor cells.

### SUMMARY

The present disclosure, in various embodiments, provides antibodies and antigen-binding fragments specific to the human GPC3 protein. Experimental testing shows that these newly identified antibodies outperformed the benchmark GC33 antibody.

In accordance with one embodiment of the present disclosure, therefore, provided is an anti-glypican 3 (GPC3) antibody or fragment thereof which has specificity to the human glypican 3 protein and comprises a heavy chain variable region (VH) comprising a VH CDR1, a VH CDR2 and a VH CDR3, and a light chain variable region (VL) comprising a VL CDR1, a VL CDR2, and a VL CDR3, wherein the VH CDR1, VH CDR2, VH CDR3, VL CDR1, VL CDR2, and VL CDR3, respectively, comprise the amino acid sequences of SEQ ID NO:71-76.

In some embodiments, the VH CDR1 comprises an amino acid sequence selected from the group consisting of SEQ ID NO:13, 25, 59 and 63; the VH CDR2 comprises an amino acid sequence selected from the group consisting of SEQ ID NO: 14, 19, 23, 26, 29, 30, 60 and 65; the VH CDR3 comprises an amino acid sequence selected from the group consisting of SEQ ID NO:15, 20, 24 and 33; the VL CDR1 comprises an amino acid sequence selected from the group consisting of SEQ ID NO:16, 21, 27 and 31; the VL CDR2 comprises an amino acid sequence selected from the group consisting of SEQ ID NO:17, 61, 64 and 66; and the VL CDR3 comprises an amino acid sequence selected from the group consisting of SEQ ID NO:18, 22, 28, 32, 34 and 62.

In some embodiments, the VH CDR1, VH CDR2, VH CDR3, VL CDR1, VL CDR2, and VL CDR3, respectively, comprise the amino acid sequences of:
SEQ ID NO:13, 19, 15, 21, 17 and 18;
SEQ ID NO: 13, 19, 20, 21, 17 and 22;
SEQ ID NO:13-18;
SEQ ID NO: 13, 23, 24, 21, 17 and 18;
SEQ ID NO:25, 26, 20, 27, 17 and 28;
SEQ ID NO:25, 29, 20, 27, 17 and 28;
SEQ ID NO:25, 30, 15, 31, 17 and 32;
SEQ ID NO:59, 60, 15, 21, 61, and 62;
SEQ ID NO:63, 19, 15, 21, 64, and 62; or
SEQ ID NO: 13, 65, 15, 21, 66, and 62.

In some embodiments, the VH CDR1 comprises an amino acid sequence selected from the group consisting of SEQ ID NO:13, 59, and 63; the VH CDR2 comprises an amino acid sequence selected from the group consisting of SEQ ID NO: 19, 60, and 65; the VH CDR3 comprises the amino acid sequence of SEQ ID NO:15; the VL CDR1 comprises the amino acid sequence of SEQ ID NO:21; the VL CDR2 comprises an amino acid sequence selected from the group consisting of SEQ ID NO:17, 61, 64 and 66; and the VL CDR3 comprises an amino acid sequence selected from the group consisting of SEQ ID NO:18 and 62.

In some embodiments, the VH CDR1, VH CDR2, VH CDR3, VL CDR1, VL CDR2, and VL CDR3, respectively, comprise the amino acid sequences of SEQ ID NO:13, 19, 15, 21, 17 and 18. In some embodiments, the VH comprises an amino acid sequence selected from the group consisting of SEQ ID NO:35-39, and the VL comprises an amino acid sequence selected from the group consisting of SEQ ID NO:40-44.

In some embodiments, the VH comprises the amino acid sequence of SEQ ID NO:36 and the VL comprises the amino acid sequence of SEQ ID NO:41. In some embodiments, the VH comprises the amino acid sequence of SEQ ID NO:37 and the VL comprises the amino acid sequence of SEQ ID NO:43.

In some embodiments, the VH comprises the amino acid sequence of SEQ ID NO:36 and the VL comprises the amino acid sequence of SEQ ID NO:42. In some embodiments, the VH comprises the amino acid sequence of SEQ ID NO:36 and the VL comprises the amino acid sequence of SEQ ID NO:43. In some embodiments, the VH comprises the amino acid sequence of SEQ ID NO:36 and the VL comprises the amino acid sequence of SEQ ID NO:44. In some embodiments, the VH comprises the amino acid sequence of SEQ ID NO:37 and the VL comprises the amino acid sequence of SEQ ID NO:41. In some embodiments, the VH comprises the amino acid sequence of SEQ ID NO:37 and the VL comprises the amino acid sequence of SEQ ID NO:42. In some embodiments, the VH comprises the amino acid sequence of SEQ ID NO:37 and the VL comprises the amino acid sequence of SEQ ID NO:44. In some embodiments, the VH comprises the amino acid sequence of SEQ ID NO:38 and the VL comprises the amino acid sequence of SEQ ID NO:41. In some embodiments, the VH comprises the amino acid sequence of SEQ ID NO:38 and the VL comprises the amino acid sequence of SEQ ID NO:42. In some embodiments, the VH comprises the amino acid sequence of SEQ ID NO:38 and the VL comprises the amino acid sequence of SEQ ID NO:43. In some embodiments, the VH comprises the amino acid sequence of SEQ ID NO:38 and the VL comprises the amino acid sequence of SEQ ID NO:44. In some embodiments, the VH comprises the amino acid sequence of SEQ ID NO:39 and the VL comprises the amino acid sequence of SEQ ID NO:41. In some embodiments, the VH comprises the amino acid sequence of SEQ ID NO:39 and the VL comprises the amino acid sequence of SEQ ID NO:42. In some embodiments, the VH comprises the amino acid sequence of SEQ ID NO:39 and the VL comprises the amino acid sequence of SEQ ID NO:43. In some embodiments, the VH comprises the amino acid sequence of SEQ ID NO:39 and the VL comprises the amino acid sequence of SEQ ID NO:44.

In some embodiments, the VH CDR1, VH CDR2, VH CDR3, VL CDR1, VL CDR2, and VL CDR3, respectively, comprise the amino acid sequences of SEQ ID NO:59, 60, 15, 21, 61, and 62. In some embodiments, the VH comprises the amino acid sequence of SEQ ID NO:53 and the VL comprises the amino acid sequence of SEQ ID NO:54.

In some embodiments, the VH CDR1, VH CDR2, VH CDR3, VL CDR1, VL CDR2, and VL CDR3, respectively, comprise the amino acid sequences of SEQ ID NO:63, 19, 15, 21, 64, and 62. In some embodiments, the VH comprises the amino acid sequence of SEQ ID NO:55 and the VL comprises the amino acid sequence of SEQ ID NO:56.

In some embodiments, the VH CDR1, VH CDR2, VH CDR3, VL CDR1, VL CDR2, and VL CDR3, respectively, comprise the amino acid sequences of SEQ ID NO:13, 65, 15, 21, 66, and 62. In some embodiments, the VH comprises the amino acid sequence of SEQ ID NO:57 and the VL comprises the amino acid sequence of SEQ ID NO:58.

In some embodiments, the VH CDR1, VH CDR2, VH CDR3, VL CDR1, VL CDR2, and VL CDR3, respectively, comprise the amino acid sequences of SEQ ID NO: 13, 19, 20, 21, 17 and 22. In some embodiments, the VH comprises an amino acid sequence selected from the group consisting of SEQ ID NO:3 and 45-50, and the VL comprises an amino acid sequence selected from the group consisting of SEQ ID NO:4, 51 and 52. In some embodiments, the VH comprises the amino acid sequence of SEQ ID NO:48 and the VL comprises the amino acid sequence of SEQ ID NO:51. In some embodiments, the VH comprises the amino acid sequence of SEQ ID NO:48 and the VL comprises the amino acid sequence of SEQ ID NO:52.

In some embodiments, the VH comprises the amino acid sequence of SEQ ID NO:45 and the VL comprises the amino acid sequence of SEQ ID NO:51. In some embodiments, the VH comprises the amino acid sequence of SEQ ID NO:45 and the VL comprises the amino acid sequence of SEQ ID NO:52. In some embodiments, the VH comprises the amino acid sequence of SEQ ID NO:46 and the VL comprises the amino acid sequence of SEQ ID NO:51. In some embodiments, the VH comprises the amino acid sequence of SEQ ID NO:46 and the VL comprises the amino acid sequence of SEQ ID NO:52. In some embodiments, the VH comprises the amino acid sequence of SEQ ID NO:47 and the VL comprises the amino acid sequence of SEQ ID NO:51. In some embodiments, the VH comprises the amino acid sequence of SEQ ID NO:47 and the VL comprises the amino acid sequence of SEQ ID NO:52. In some embodiments, the VH comprises the amino acid sequence of SEQ ID NO:49 and the VL comprises the amino acid sequence of SEQ ID NO:51. In some embodiments, the VH comprises the amino acid sequence of SEQ ID NO:49 and the VL comprises the amino acid sequence of SEQ ID NO:52. In some embodiments, the VH comprises the amino acid sequence of SEQ ID NO:50 and the VL comprises the amino acid sequence of SEQ ID NO:51. In some embodiments, the VH comprises the amino acid sequence of SEQ ID NO:50 and the VL comprises the amino acid sequence of SEQ ID NO:52.

In some embodiments, the VH CDR1, VH CDR2, VH CDR3, VL CDR1, VL CDR2, and VL CDR3, respectively, comprise the amino acid sequences of SEQ ID NO:13-18. In some embodiments, the VH comprises the amino acid sequence of SEQ ID NO:1, and the VL comprises the amino acid sequence of SEQ ID NO:2.

In some embodiments, the VH CDR1, VH CDR2, VH CDR3, VL CDR1, VL CDR2, and VL CDR3, respectively, comprise the amino acid sequences of SEQ ID NO:13, 23, 24, 21, 17 and 18. In some embodiments, the VH comprises the amino acid sequence of SEQ ID NO:5, and the VL comprises the amino acid sequence of SEQ ID NO:6.

In some embodiments, the VH CDR1, VH CDR2, VH CDR3, VL CDR1, VL CDR2, and VL CDR3, respectively, comprise the amino acid sequences of SEQ ID NO:25, 26, 20, 27, 17 and 28. In some embodiments, the VH comprises the amino acid sequence of SEQ ID NO:7, and the VL comprises the amino acid sequence of SEQ ID NO:8.

In some embodiments, the VH CDR1, VH CDR2, VH CDR3, VL CDR1, VL CDR2, and VL CDR3, respectively, comprise the amino acid sequences of SEQ ID NO:25, 29, 20, 27, 17 and 28. In some embodiments, the VH comprises the amino acid sequence of SEQ ID NO:9, and the VL comprises the amino acid sequence of SEQ ID NO:10.

In some embodiments, the VH CDR1, VH CDR2, VH CDR3, VL CDR1, VL CDR2, and VL CDR3, respectively, comprise the amino acid sequences of SEQ ID NO:25, 30, 15, 31, 17 and 32. In some embodiments, the VH comprises the amino acid sequence of SEQ ID NO:11, and the VL comprises the amino acid sequence of SEQ ID NO:12.

In some embodiments, the VH CDR1 comprises the amino acid sequence of SEQ ID NO:13; the VH CDR2 comprises the amino acid sequence of SEQ ID NO:19; the VH CDR3 comprises an amino acid sequence selected from the group consisting of SEQ ID NO:15, 20, 24, and 33; the VL CDR1 comprises the amino acid sequence of SEQ ID NO:21; the VL CDR2 comprises the amino acid sequence of SEQ ID NO:17; and the VL CDR3 comprises an amino acid sequence selected from the group consisting SEQ ID NO:18, 22, 28, 32 and 34.

In some embodiments, the antibody or fragment thereof is humanized.

Also provided, in one embodiment, is an antibody-drug conjugate, characterized in that the antibody-drug conjugate comprises: (a) an antibody or fragment thereof of the present disclosure; and (b) a conjugation moiety conjugated to the antibody or fragment, wherein the conjugation moiety is selected from: a detectable marker, a drug, a toxin, a cytokine, a radionuclide, an enzyme, or a combination thereof.

Also provided, in one embodiment, is a multispecific antibody comprising the antibody of the present disclosure and one or more second antibody or antigen-binding fragment having binding specificity to a target antigen that is not GPC3.

Also provided is a chimeric antigen receptor (CAR) comprising the antibody or fragment thereof of the present disclosure, a transmembrane domain, a costimulatory domain, and a CD3ξ intracellular domain.

In another embodiment, provided is a polynucleotide encoding the antibody or fragment thereof of the present disclosure, a cell comprising the polynucleotide, and a composition comprising the antibody or fragment thereof and a pharmaceutically acceptable carrier. Also provided are methods and uses for treating cancer with the antibody or fragment thereof.

### BRIEF DESCRIPTION OF THE DRAWINGS

**FIG. 1** shows the binding affinity of the 52H5D3B8 antibody to the GPC3 protein (human GPC3-his tag) as measured by Biacore.
**FIG. 2A-B** show that the affinity of 52H5D3B8 antibody is higher than GC33 for binding to GPC3-CHOK1 cells, HEPG2 cells, Hep3B cells or Huh-7 cells.
**FIG. 3** shows the ELISA binding activity of 52H5D3B8 bound to human, mouse and cynomolgus GPC3.
**FIG. 4** shows that the affinity of the 52H5D3B8-6# chimeric monoclonal antibodies (mAbs) for binding to GPC3-CHOK1 cells is higher than other optimized 52H5D3B8 chimeric mAbs.
**FIG. 5** shows that the affinity of the 52H5D3B8-6# chimeric mAb for binding to GPC3-CHOK1 cells, Hep3B cells and Huh-7 cells is the highest among 52H5D3B8 chimeric mAb and GC33 antibodies.
**FIG. 6** shows the binding affinity of the 52H5D3B8-6# antibody to the GPC3 protein (human GPC3-his tag) as measured by Biacore 8K to chimeric antibody.
**FIG. 7** shows the EC50 of each humanized antibody on GPC3-CHOK1 cells, HEPG2 cells, Hep3B cells and Huh-7 cells.
**FIG. 8** shows that the EC50 of each humanized antibody on GPC3-CHOK1 cells, HEPG2 cells, Hep3B cells and Huh-7 cells.
**FIG. 9** shows the binding of 52H5D3B8-6#-VH+VL, 52H5D3B8-6#- VHI + VL 1, 52H5D3B8-6#-VH1+VL3, 52H5D3B8-6#- VHI + VL4, 52H5D3B8-6#-VH2+VL4,52H5D3B8-6#-VH3+VL1 to recombinant GPC3 protein (human GPC3-his tag) as tested by Biacore using a capture method.
**FIG. 10** shows the results of antibody binding to human GPC3-overexpressing CHOK1 cells and HEPG2 cells.
**FIG. 11** shows the results of binding to human GPC3-overexpressing CHOK1 cells for the affinity-matured antibodies.
**FIG. 12** shows the results of the antibodies inducing GPC3 antibody mediated ADCC signaling.
**FIG. 13** shows that the antibodies activated NK cell function.
**FIG. 14** shows degranulation of NK cells stimulated by the antibodies.
**FIG. 15** shows cytokine production in stimulated NK cells.
**FIG. 16** shows that the binding efficiency of all candidate humanized antibodies is comparable to the chimeric antibody on GPC3-CHOK1 cells and better than the chimeric antibody on Huh-7 cells.
**FIG. 17** shows the results of antibody binding to human GPC3-overexpressing CHOK1 cells, Huh-7 cells and HEPG2 cells.
**FIG. 18** shows the results of antibody internalization in HEPG2 cells.

### DETAILED DESCRIPTION

### Definitions

It is to be noted that the term "a" or "an" entity refers to one or more of that entity; for example, "an antibody," is understood to represent one or more antibodies. As such, the terms "a" (or "an"), "one or more," and "at least one" can be used interchangeably herein.

As used herein, an "antibody" or "antigen-binding polypeptide" refers to a polypeptide or a polypeptide complex that specifically recognizes and binds to an antigen. An antibody can be a whole antibody and any antigen binding fragment or a single chain thereof. Thus the term "antibody" includes any protein or peptide containing molecule that comprises at least a portion of an immunoglobulin molecule having biological activity of binding to the antigen. Examples of such include, but are not limited to a complementarity determining region (CDR) of a heavy or light chain or a ligand binding portion thereof, a heavy chain or light chain variable region, a heavy chain or light chain constant region, a framework (FR) region, or any portion thereof, or at least one portion of a binding protein.

The terms "antibody fragment" or "antigen-binding fragment", as used herein, is a portion of an antibody such as F(ab')2, F(ab)2, Fab', Fab, Fv, scFv and the like. Regardless of structure, an antibody fragment binds with the same antigen that is recognized by the intact antibody. The term "antibody fragment" includes aptamers, spiegelmers, and diabodies. The term "antibody fragment" also includes any synthetic or genetically engineered protein that acts like an antibody by binding to a specific antigen to form a complex.

The term antibody encompasses various broad classes of polypeptides that can be distinguished biochemically. Those skilled in the art will appreciate that heavy chains are classified as gamma, mu, alpha, delta, or epsilon (γ, µ, α, δ, ε) with some subclasses among them (e.g., γ1-γ4). It is the nature of this chain that determines the "class" of the antibody as IgG, IgM, IgA IgG, or IgE, respectively.

The immunoglobulin subclasses (isotypes) e.g., IgG1, IgG2, IgG3, IgG4, IgG5, etc. are well characterized and are known to confer functional specialization. Modified versions of each of these classes and isotypes are readily discernable to the skilled artisan in view of the instant disclosure and, accordingly, are within the scope of the instant disclosure. All immunoglobulin classes are clearly within the scope of the present disclosure, the following discussion will generally be directed to the IgG class of immunoglobulin molecules. With regard to IgG, a standard immunoglobulin molecule comprises two identical light chain polypeptides of molecular weight approximately 23,000 Daltons, and two identical heavy chain polypeptides of molecular weight 53,000-70,000. The four chains are typically joined by disulfide bonds in a "Y" configuration wherein the light chains bracket the heavy chains starting at the mouth of the "Y" and continuing through the variable region.

Antibodies, antigen-binding polypeptides, variants, or derivatives thereof of the disclosure include, but are not limited to, polyclonal, monoclonal, multispecific, human, humanized, primatized, or chimeric antibodies, single chain antibodies, epitope-binding fragments, e.g., Fab, Fab' and F(ab')2, Fd, Fvs, single-chain Fvs (scFv), single-chain antibodies, disulfide-linked Fvs (sdFv), fragments comprising either a VK or VH domain, fragments produced by a Fab expression library, and anti-idiotypic (anti-Id) antibodies (including, e.g., anti-Id antibodies to the antibodies disclosed herein). Immunoglobulin or antibody molecules of the disclosure can be of any type (e.g., IgG, IgE, IgM, IgD, IgA, and IgY), class (e.g., IgGl, IgG2, IgG3, IgG4, IgAl and IgA2) or subclass of immunoglobulin molecule.

As used herein, the term "chimeric antibody" will be held to mean any antibody wherein the immunoreactive region or site is obtained or derived from a first species and the constant region (which may be intact, partial or modified in accordance with the instant disclosure) is obtained from a second species. In certain embodiments the target binding region or site will be from a non-human source (e.g. mouse or primate) and the constant region is human.

Antibodies disclosed herein can be from any animal origin including birds and mammals. Preferably, the antibodies are human, murine, donkey, rabbit, goat, guinea pig, camel, llama, horse, or chicken antibodies. In some embodiments, the variable region may be condricthoid in origin (e.g., from sharks).

As used herein, the term "recombinant" as it pertains to polypeptides or polynucleotides intends a form of the polypeptide or polynucleotide that does not exist naturally, a non-limiting example of which can be created by combining polynucleotides that would not normally occur together.

Hybridoma technology can be performed under conditions of different "stringency". In general, a low stringency hybridization reaction is carried out at about 40°C in about 10 x SSC or a solution of equivalent ionic strength/temperature. A moderate stringency hybridization is typically performed at about 50°C in about 6 x SSC, and a high stringency hybridization reaction is generally performed at about 60°C in about 1 x SSC. Hybridization reactions can also be performed under "physiological conditions" which is well known to one of skill in the art. A nonlimiting example of a physiological condition is the temperature, ionic strength, pH and concentration of Mg2+ normally found in a cell.

### GPC-3 Antibodies

As demonstrated in the appended experimental examples, the instant inventors were able to generate potent murine anti-GPC3 antibodies 18C1D9, 52H5D3B8, 163E7D12, 172B4E9, 152E9F7 and 171F9C5 **(Table 1).** As shown in Example 3, for instance, the 52H5D3B8 antibody exhibited higher cell-based binding efficiency than GC33, a leading anti-GPC3 antibody under clinical development. In addition, experimental data show that these new antibodies have higher activity in inducing cellular internationalization. Such a property makes these new antibodies particularly suitable for use in, e.g., antibody-drug conjugates (ADC).

Moreover, through amino acid substitutions in the VH and VL CDR3, the sequences of the 52H5D3B8 antibody were further optimized. One of such optimized antibodies, 52H5D3B8-6#, for instance, bound to GPC3-CHOK1 cells, Hep3B cells and Huh-7 cells with higher affinity than both the parental 52H5D3B8 chimeric mAb and the reference GC33 antibody.

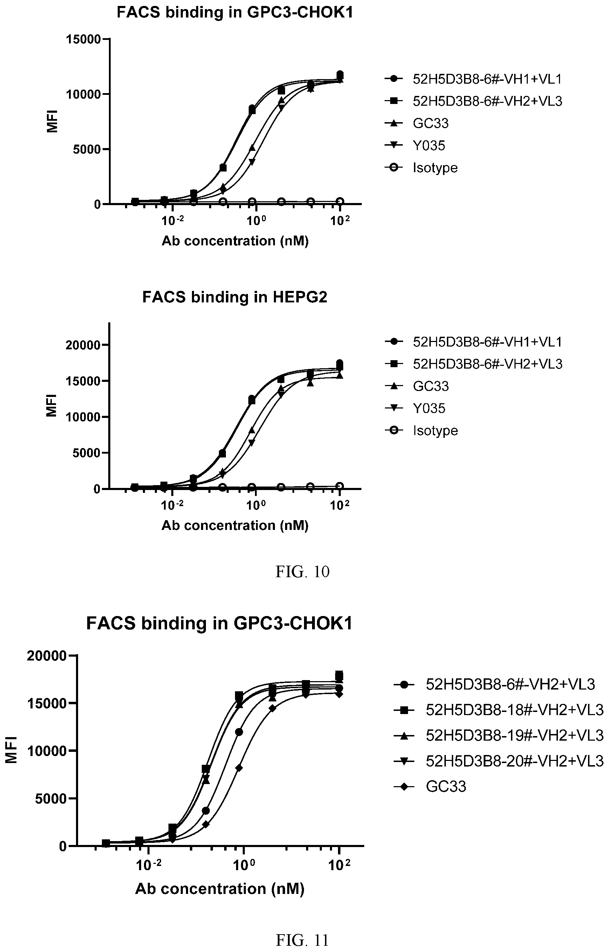

Humanized versions of the 52H5D3B8-6# antibody were generated and compared to GC33 as well as another benchmark antibody, Y035 (as disclosed in US patent application US20190046659). Interestingly, as shown **FIG. 10** **and Table 14,** humanized antibodies 52H5D3B8-6#- VHI + VL 1 and 52H5D3B8-6#-VH2+VL3 retained the high affinity of the parental antibody, and remarkably outperformed both GC33 and Y035.

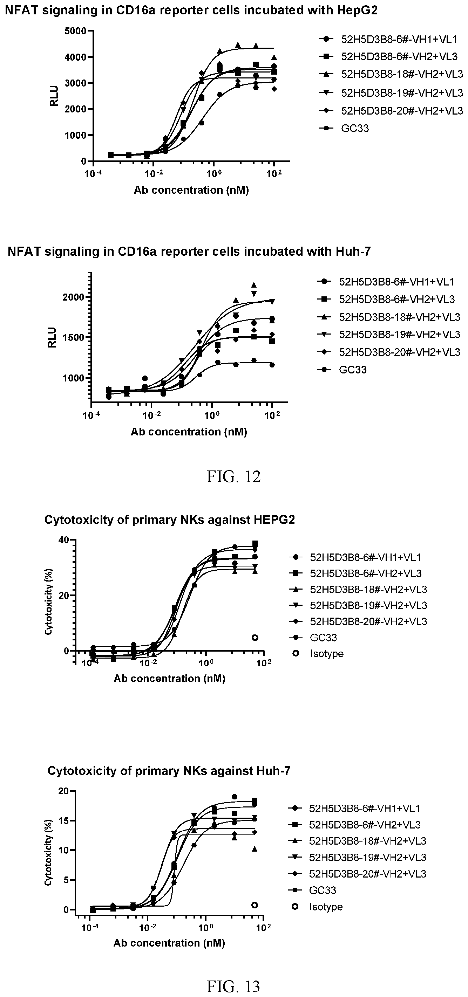

The humanized antibodies were subjected to further affinity maturation in an effort to further enhance binding efficiency. As shown in **FIG. 11** and **Table 16,** encouragingly, all of the affinity maturated antibodies exhibited enhanced binding efficiency as compared to the parental antibody (52H5D3B8-6#-VH2+VL3) and benchmark antibody GC33. Also surprisingly, when targeting cells with moderate GPC3 expression levels, the humanized antibodies exhibited considerably higher ADCC signaling potency over GC33 **(****FIG. 12** and **Table 17).**

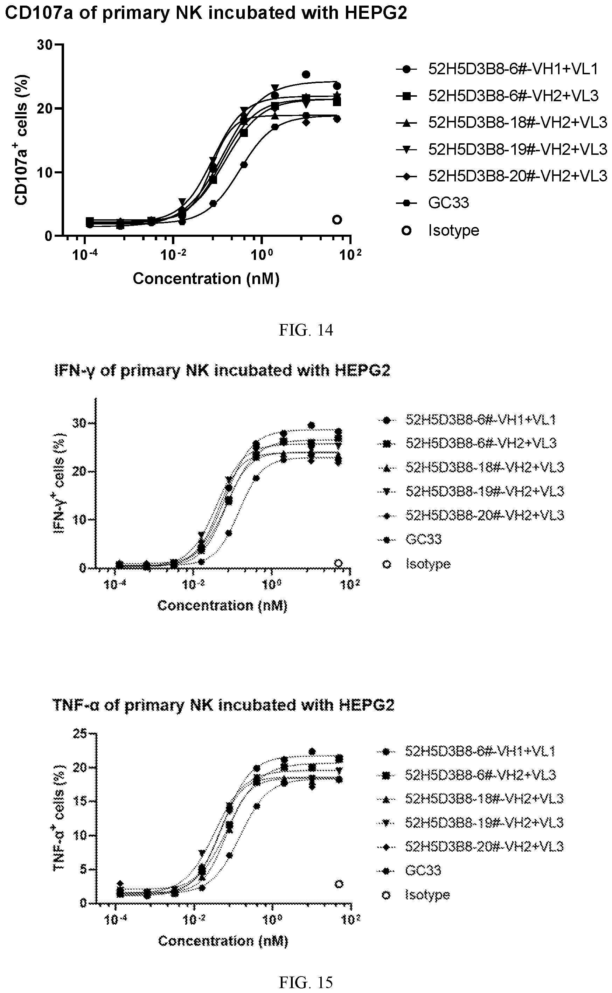

These humanized and affinity maturated antibodies were further tested for their ability to activate NK cells in the presence of GPC3-expressing cells. As shown in **FIG. 13** and **Table 18,** all of these new antibodies had higher activity than GC33. They induced higher production of IFN-γ and the TNF-α in NK cells than GC33 **(****FIG. 15****).**

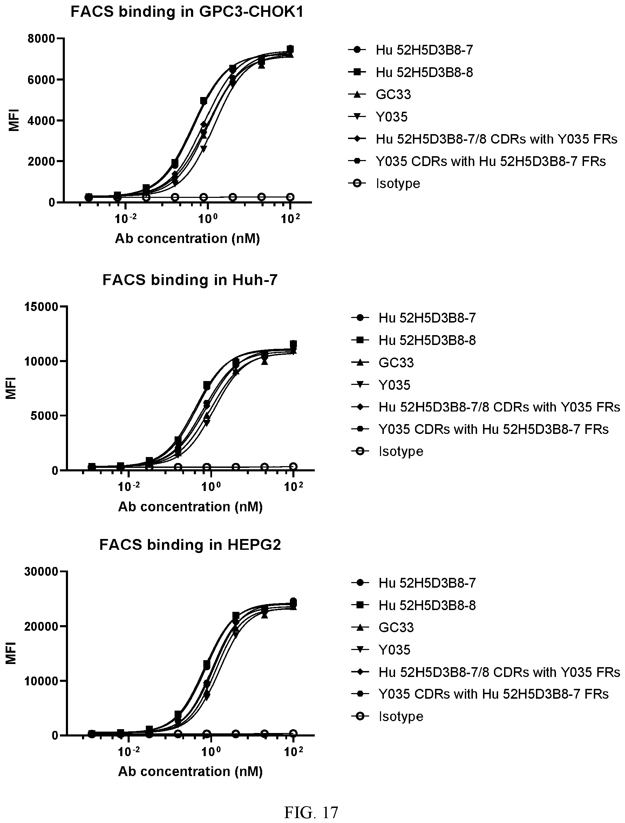

The parental antibody 52H5D3B8 was also subjected to humanization, and two of the humanized ones, Hu 52H5D3B8-7 and Hu 52H5D3B8-8, were tested for their binding activity and internalization activity, with GC33 and Y035 as references. As shown in **FIG. 17-18** and **Tables 24-25,** these humanized antibodies outperformed both references. Quite interestingly, when the CDRs of Hu 52H5D3B8-7/8 were inserted to the framework of Y035 or the framework regions of Hu 52H5D3B8-7/8 were used to support the CDRs of Y035, the resulting grafted antibodies were all better-performing than Y035 **(Table 24),** underscoring the excellence of both the CDRs and framework sequences of these new antibodies.

It is observed that these antibodies and their CDR sequences are highly homologous to one another, suggesting that the CDR sequences can be interchangeable (see **Table A).**

**Table A. CDR sequences**

| **CDR** | **Sequence** | **SEQ ID NO:** |
|---|---|---|
| CDRH1 | **X₁**YE**X₂**H (X₁ is D/R/G, X₂ is I or M) | 71 |
| | DYEMH | 13 |
| | DYEIH | 25 |
| | RYEMH | 59 |
| | GYEMH | 63 |
| CDRH2 | AI**X₁**P**X₂X₃X₄X₅**TAY**X₆X₇X₈**FKG (X₁ is D/G/H, X₂ is A/E/G, X₃ is S/T, X₄ is D/G, X₅ is D/G/N/S, X₆ is N/S/T, X₇ is Q/S, X₈ is R/K/L) | 72 |
| | AIDPETGGTAYNQKFKG | 14 |
| | AIHPGSGGTAYNQKFKG | 19 |
| | AIGPETGSTAYNQRFKG | 23 |
| | AIDPATGDTAYTQKFKG | 26 |
| | AIDPETGDTAYTQKFKG | 29 |
| | AIDPETDNTAYSQKFKG | 30 |
| | AIHPGSGGTAYNSKFKG | 60 |
| | AIHPGSGGTAYNQLFKG | 65 |
| CDRH3 | **X₁**YS**X₂**AY (X₁ is F/Y, X₂ is F/Y) | 73 |
| | YYSYAY | 15 |
| | FYSYAY | 20 |
| | YYSFAY | 24 |
| | FYSFAY | 33 |
| CDRL1 | RS**X₁**QS**X₂**VH**X₃**NG**X₄**TYL**X₅** (X₁ is R/S, X₂ is L/P, X₃ is S/R/T, X₄ is H/N, X₅ is H/Q) | 74 |
| | RSRQSLVHSNGNTYLQ | 16 |
| | RSSQSLVHSNGNTYLQ | 21 |
| | RSSQSLVHTNGHTYLQ | 27 |
| | RSSQSPVHRNGNTYLH | 31 |
| CDRL2 | KVSNRX₁X₂ (X₁ is F/Y, X₂ is S/A/P) | 75 |
| | KVSNRFS | 17 |
| | KVSNRYS | 61 |
| | KVSNRFA | 64 |
| | KVSNRFP | 66 |
| CDRL3 | **X₁X₂X₃X₄**HVPYT (X₁ is F/S/V, X₂ is Q/E, X₃ is S/T, X₄ is I/T) | 76 |
| | FQSIHVPYT | 18 |
| | SQSIHVPYT | 22 |
| | SQSTHVPYT | 28 |
| | SQTTHVPYT | 32 |
| | FQSTHVPYT | 34 |
| | VESIHVPYT | 62 |

Accordingly, in one embodiment, the present disclosure provides an anti-glypican 3 antibody that includes a VH (heavy chain variable region) and a VL (light chain variable region). The VH and VL regions include VH CDR1, VH CDR2, VH CDR3, VL CDR1, VL CDR2, and VL CDR3, such as those illustrated in **Tables A, 1A-1F, 5A-5O, 8A and 8B.**

In some embodiments, the VH CDR1 includes the amino acid sequence of SEQ ID NO:71, the VH CDR2 includes the amino acid sequence of SEQ ID NO:72, the VH CDR3 includes the amino acid sequence of SEQ ID NO:73, the VL CDR1 includes the amino acid sequence of SEQ ID NO:74, the VL CDR2 includes the amino acid sequence of SEQ ID NO:75, and the VL CDR3 includes the amino acid sequence of SEQ ID NO:76.

SEQ ID NO:71 is **X₁**YE**X₂**H, where X₁ can be D, R or G, X₂ can be I or M. SEQ ID NO:72 is AI**X₁PX₂X₃X₄X₅**TAY**X₆X₇X₈**FKG, where X₁ can be D, G, or H, X₂ can be A, E or G, X₃ can be S or T, X₄ can be D or G, X₅ can be D, G, N or S, X₆ can be N, S or T, X₇ can be Q or S, and Xs can be R, K or L. SEQ ID NO:73 is **X₁**YS**X₂**AY, where X₁ can be F or Y, and X₂ can be F or Y SEQ ID NO:74 is RS**X₁**QS**X₂**VH**X₃**NG**X₄**TYL**X₅**, where X₁ can be R or S, X₂ can be L or P, X₃ can be S, R or T, X₄ can be H or N, and X₅ can be H or Q. SEQ ID NO:75 is KVSNRX₁X₂, where X₁ can be F or Y, and X₂ can be S, A or P. SEQ ID NO:76 is **X₁X₂X₃X₄**HVPYT, where X₁ can be F, S or V, X₂ can be Q or E, X₃ can be S or T, and X₄ can be I or T.

In some embodiments, the VH CDR1 includes an amino acid sequence selected from the group consisting of SEQ ID NO: 13 and 25; the VH CDR2 comprises an amino acid sequence selected from the group consisting of SEQ ID NO: 14, 19, 23, 26, 29 and 30; the VH CDR3 comprises an amino acid sequence selected from the group consisting of SEQ ID NO: 15, 20, 24 and 33; the VL CDR1 comprises an amino acid sequence selected from the group consisting of SEQ ID NO: 16, 21, 27 and 31; the VL CDR2 comprises the amino acid sequence of SEQ ID NO: 17; and the VL CDR3 comprises an amino acid sequence selected from the group consisting of SEQ ID NO: 18, 22, 28, 32 and 34.

In some embodiments, the VH CDR1 comprises an amino acid sequence selected from the group consisting of SEQ ID NO: 13, 25, 59 and 63; the VH CDR2 comprises an amino acid sequence selected from the group consisting of SEQ ID NO: 14, 19, 23, 26, 29, 30, 60 and 65; the VH CDR3 comprises an amino acid sequence selected from the group consisting of SEQ ID NO: 15, 20, 24 and 33; the VL CDR1 comprises an amino acid sequence selected from the group consisting of SEQ ID NO: 16, 21, 27 and 31; the VL CDR2 comprises an amino acid sequence selected from the group consisting of SEQ ID NO: 17, 61, 64 and 66; and the VL CDR3 comprises an amino acid sequence selected from the group consisting of SEQ ID NO: 18, 22, 28, 32, 34 and 62.

Also provided, in some embodiments, are anti-GPC3 antibodies and antigen binding fragments that compete with any of the antibodies disclosed herein in binding to human GPC3. Also provided, in some embodiments, are anti-GPC3 antibodies and antigen binding fragments that bind to the same epitope as any of the antibodies disclosed herein. Also provided, in some embodiments, are anti-GPC3 antibodies and antigen binding fragments that included the VH and VL CDR1, CDR2, and CDR3 of the antibodies disclosed herein.

In some embodiments, provided is an antibody or antigen-binding fragment that includes the CDRs of antibody 52H5D3B8-6# or its humanized/affinity maturated versions. In some embodiments, the VH CDR1 comprises an amino acid sequence selected from the group consisting of SEQ ID NO: 13, 59, and 63; the VH CDR2 comprises an amino acid sequence selected from the group consisting of SEQ ID NO: 19, 60, and 65; the VH CDR3 comprises the amino acid sequence of SEQ ID NO:15; the VL CDR1 comprises the amino acid sequence of SEQ ID NO:21; the VL CDR2 comprises an amino acid sequence selected from the group consisting of SEQ ID NO:17, 61, 64 and 66; and the VL CDR3 comprises an amino acid sequence selected from the group consisting of SEQ ID NO:18 and 62. In some embodiments, the VH CDR1, VH CDR2, VH CDR3, VL CDR1, VL CDR2, and VL CDR3, respectively, comprise the amino acid sequences of SEQ ID NO: 13, 19, 15, 21, 17 and 18. In some embodiments, the VH comprises an amino acid sequence selected from the group consisting of SEQ ID NO:35-39, and the VL comprises an amino acid sequence selected from the group consisting of SEQ ID NO:40-44.

Example humanized antibodies or antigen-binding fragment derived from 52H5D3B8-6# include those illustrated in **Table 8A.** Examples include those that have a VH of SEQ ID NO:36 and a VL of SEQ ID NO:41, a VH of SEQ ID NO:36 and a VL of SEQ ID NO:42, a VH of SEQ ID NO:36 and a VL of SEQ ID N0:43, a VH of SEQ ID NO:36 and a VL of SEQ ID NO:44, VH of SEQ ID NO:37 and a VL of SEQ ID NO:41, a VH of SEQ ID NO:37 and a VL of SEQ ID NO:42, a VH of SEQ ID NO:37 and a VL of SEQ ID NO:43, a VH of SEQ ID NO:37 and a VL of SEQ ID NO:44, VH of SEQ ID NO:38 and a VL of SEQ ID NO:41, a VH of SEQ ID NO:38 and a VL of SEQ ID N0:42, a VH of SEQ ID NO:38 and a VL of SEQ ID N0:43, or a VH of SEQ ID NO:38 and a VL of SEQ ID N0:44.

In some embodiments, the VH comprises the amino acid sequence of SEQ ID NO:36 and the VL comprises the amino acid sequence of SEQ ID NO:41. In some embodiments, the VH comprises the amino acid sequence of SEQ ID NO:37 and the VL comprises the amino acid sequence of SEQ ID NO:43.

In some embodiments, the VH comprises the amino acid sequence of SEQ ID NO:36 and the VL comprises the amino acid sequence of SEQ ID NO:42. In some embodiments, the VH comprises the amino acid sequence of SEQ ID NO:36 and the VL comprises the amino acid sequence of SEQ ID NO:43. In some embodiments, the VH comprises the amino acid sequence of SEQ ID NO:36 and the VL comprises the amino acid sequence of SEQ ID NO:44. In some embodiments, the VH comprises the amino acid sequence of SEQ ID NO:37 and the VL comprises the amino acid sequence of SEQ ID NO:41. In some embodiments, the VH comprises the amino acid sequence of SEQ ID NO:37 and the VL comprises the amino acid sequence of SEQ ID NO:42. In some embodiments, the VH comprises the amino acid sequence of SEQ ID NO:37 and the VL comprises the amino acid sequence of SEQ ID NO:44. In some embodiments, the VH comprises the amino acid sequence of SEQ ID NO:38 and the VL comprises the amino acid sequence of SEQ ID NO:41. In some embodiments, the VH comprises the amino acid sequence of SEQ ID NO:38 and the VL comprises the amino acid sequence of SEQ ID NO:42. In some embodiments, the VH comprises the amino acid sequence of SEQ ID NO:38 and the VL comprises the amino acid sequence of SEQ ID NO:43. In some embodiments, the VH comprises the amino acid sequence of SEQ ID NO:38 and the VL comprises the amino acid sequence of SEQ ID NO:44. In some embodiments, the VH comprises the amino acid sequence of SEQ ID NO:39 and the VL comprises the amino acid sequence of SEQ ID NO:41. In some embodiments, the VH comprises the amino acid sequence of SEQ ID NO:39 and the VL comprises the amino acid sequence of SEQ ID NO:42. In some embodiments, the VH comprises the amino acid sequence of SEQ ID NO:39 and the VL comprises the amino acid sequence of SEQ ID NO:43. In some embodiments, the VH comprises the amino acid sequence of SEQ ID NO:39 and the VL comprises the amino acid sequence of SEQ ID NO:44.

Affinity-maturated versions of these antibodies are also provided, such as52H5D3B8-18#-VH2+VL3, 52H5D3B8-19#-VH2+VL3, and 52H5D3B8-20#-VH2+VL3 **(Table 15).** In some embodiments, the VH CDR1, VH CDR2, VH CDR3, VL CDR1, VL CDR2, and VL CDR3, respectively, comprise the amino acid sequences of SEQ ID NO:59, 60, 15, 21, 61, and 62. In some embodiments, the VH comprises the amino acid sequence of SEQ ID NO:53 and the VL comprises the amino acid sequence of SEQ ID NO:54.

In some embodiments, the VH CDR1, VH CDR2, VH CDR3, VL CDR1, VL CDR2, and VL CDR3, respectively, comprise the amino acid sequences of SEQ ID NO:63, 19, 15, 21, 64, and 62. In some embodiments, the VH comprises the amino acid sequence of SEQ ID NO:55 and the VL comprises the amino acid sequence of SEQ ID NO:56.

In some embodiments, the VH CDR1, VH CDR2, VH CDR3, VL CDR1, VL CDR2, and VL CDR3, respectively, comprise the amino acid sequences of SEQ ID NO: 13, 65, 15, 21, 66, and 62. In some embodiments, the VH comprises the amino acid sequence of SEQ ID NO:57 and the VL comprises the amino acid sequence of SEQ ID NO:58.

In some embodiments, provided is an antibody or antigen-binding fragment that includes the CDRs of antibody 52H5D3B8. In some embodiments, the VH CDR1, VH CDR2, VH CDR3, VL CDR1, VL CDR2, and VL CDR3, respectively, comprise the amino acid sequences of SEQ ID NO: 13, 19, 20, 21, 17 and 22. In some embodiments, the VH comprises an amino acid sequence selected from the group consisting of SEQ ID NO:3 and 45-50, and the VL comprises an amino acid sequence selected from the group consisting of SEQ ID NO:4, 51 and 52.

Example humanized antibodies or antigen-binding fragment derived from 52H5D3B8 include those illustrated in **Table 19.** Examples include those that have a VH of SEQ ID NO:45 and a VL of SEQ ID NO:51, a VH of SEQ ID NO:46 and a VL of SEQ ID NO:51, a VH of SEQ ID NO:47 and a VL of SEQ ID NO:51, a VH of SEQ ID NO:48 and a VL of SEQ ID NO:51, a VH of SEQ ID NO:49 and a VL of SEQ ID NO:51, a VH of SEQ ID NO:50 and a VL of SEQ ID NO:51, a VH of SEQ ID NO:45 and a VL of SEQ ID NO:52, a VH of SEQ ID NO:46 and a VL of SEQ ID NO:52, a VH of SEQ ID NO:47 and a VL of SEQ ID NO:52, a VH of SEQ ID NO:48 and a VL of SEQ ID NO:52, a VH of SEQ ID NO:49 and a VL of SEQ ID NO:52, or a VH of SEQ ID NO:50 and a VL of SEQ ID NO:52.

In some embodiments, the VH comprises the amino acid sequence of SEQ ID NO:48 and the VL comprises the amino acid sequence of SEQ ID NO:51. In some embodiments, the VH comprises the amino acid sequence of SEQ ID NO:48 and the VL comprises the amino acid sequence of SEQ ID NO:52.

In some embodiments, provided is an antibody or antigen-binding fragment that is derived from antibody 52H5D3B8, such as those having alternative VH CDR3 and VL CDR3 as illustrated in **Tables 5A-5O.** In some embodiments, the VH CDR1 comprises the amino acid sequence of SEQ ID NO:13; the VH CDR2 comprises the amino acid sequence of SEQ ID NO:19; the VH CDR3 comprises an amino acid sequence selected from the group consisting of SEQ ID NO:15, 20, 24, and 33; the VL CDR1 comprises the amino acid sequence of SEQ ID NO:21; the VL CDR2 comprises the amino acid sequence of SEQ ID NO:17; and the VL CDR3 comprises an amino acid sequence selected from the group consisting SEQ ID NO:18, 22, 28, 32 and 34.

In some embodiments, the VH comprises the amino acid sequence of SEQ ID NO:35 and the VL comprises the amino acid sequence of SEQ ID NO:4. In some embodiments, the VH comprises the amino acid sequence of SEQ ID NO:67 and the VL comprises the amino acid sequence of SEQ ID NO:4. In some embodiments, the VH comprises the amino acid sequence of SEQ ID NO:3 and the VL comprises the amino acid sequence of SEQ ID NO:40. In some embodiments, the VH comprises the amino acid sequence of SEQ ID NO:3 and the VL comprises the amino acid sequence of SEQ ID NO:68. In some embodiments, the VH comprises the amino acid sequence of SEQ ID NO:69 and the VL comprises the amino acid sequence of SEQ ID NO:4. In some embodiments, the VH comprises the amino acid sequence of SEQ ID NO:35 and the VL comprises the amino acid sequence of SEQ ID NO:68. In some embodiments, the VH comprises the amino acid sequence of SEQ ID NO:67 and the VL comprises the amino acid sequence of SEQ ID NO:40. In some embodiments, the VH comprises the amino acid sequence of SEQ ID NO:67 and the VL comprises the amino acid sequence of SEQ ID NO:68. In some embodiments, the VH comprises the amino acid sequence of SEQ ID NO:3 and the VL comprises the amino acid sequence of SEQ ID NO:70. In some embodiments, the VH comprises the amino acid sequence of SEQ ID NO:67 and the VL comprises the amino acid sequence of SEQ ID NO:70. In some embodiments, the VH comprises the amino acid sequence of SEQ ID NO:35 and the VL comprises the amino acid sequence of SEQ ID NO:70. In some embodiments, the VH comprises the amino acid sequence of SEQ ID NO:69 and the VL comprises the amino acid sequence of SEQ ID NO:68. In some embodiments, the VH comprises the amino acid sequence of SEQ ID NO:69 and the VL comprises the amino acid sequence of SEQ ID NO:40. In some embodiments, the VH comprises the amino acid sequence of SEQ ID NO:69 and the VL comprises the amino acid sequence of SEQ ID NO:70.

In some embodiments, provided is an antibody or antigen-binding fragment that includes the CDRs of antibody 18C1D9. In some embodiments, the VH CDR1, VH CDR2, VH CDR3, VL CDR1, VL CDR2, and VL CDR3, respectively, comprise the amino acid sequences of SEQ ID NO: 13-18. In some embodiments, the VH comprises the amino acid sequence of SEQ ID NO: 1, and the VL comprises the amino acid sequence of SEQ ID NO:2.

In some embodiments, provided is an antibody or antigen-binding fragment that includes the CDRs of antibody 163E7D12. In some embodiments, the VH CDR1, VH CDR2, VH CDR3, VL CDR1, VL CDR2, and VL CDR3, respectively, comprise the amino acid sequences of SEQ ID NO: 13, 23, 24, 21, 17 and 18. In some embodiments, the VH comprises the amino acid sequence of SEQ ID NO:5, and the VL comprises the amino acid sequence of SEQ ID NO:6.

In some embodiments, provided is an antibody or antigen-binding fragment that includes the CDRs of antibody 172B4E9. In some embodiments, the VH CDR1, VH CDR2, VH CDR3, VL CDR1, VL CDR2, and VL CDR3, respectively, comprise the amino acid sequences of SEQ ID NO:25, 26, 20, 27, 17 and 28. In some embodiments, the VH comprises the amino acid sequence of SEQ ID NO:7, and the VL comprises the amino acid sequence of SEQ ID NO:8.

In some embodiments, provided is an antibody or antigen-binding fragment that includes the CDRs of antibody 152E9F7. In some embodiments, the VH CDR1, VH CDR2, VH CDR3, VL CDR1, VL CDR2, and VL CDR3, respectively, comprise the amino acid sequences of SEQ ID NO:25, 29, 20, 27, 17 and 28. In some embodiments, the VH comprises the amino acid sequence of SEQ ID NO:9, and the VL comprises the amino acid sequence of SEQ ID NO:10.

In some embodiments, provided is an antibody or antigen-binding fragment that includes the CDRs of antibody 171F9C5. In some embodiments, the VH CDR1, VH CDR2, VH CDR3, VL CDR1, VL CDR2, and VL CDR3, respectively, comprise the amino acid sequences of SEQ ID NO:25, 30, 15, 31, 17 and 32. In some embodiments, the VH comprises the amino acid sequence of SEQ ID NO:11, and the VL comprises the amino acid sequence of SEQ ID NO:12.

### Antibody-Drug Conjugates

Preliminary data show that these new antibodies have higher activity in inducing cellular internationalization. Such a property makes these new antibodies particularly suitable for use in, e.g., antibody-drug conjugates (ADC).

In some embodiments, the antibodies or fragments may be conjugated to therapeutic agents, prodrugs, peptides, proteins, enzymes, viruses, lipids, biological response modifiers, pharmaceutical agents, or PEG.

In one embodiment, the antibodies or fragments of the disclosure are covalently attached to a drug moiety. The drug moiety may be, or be modified to include, a group reactive with a conjugation point on the antibody. For example, a drug moiety can be attached by alkylation (e.g., at the epsilon-amino group lysines or the N-terminus of antibodies), reductive amination of oxidized carbohydrate, transesterification between hydroxyl and carboxyl groups, amidation at amino groups or carboxyl groups, and conjugation to thiols.

In some embodiments, the number of drug moieties, p, conjugated per antibody molecule ranges from an average of 1 to 8; 1 to 7, 1 to 6, 1 to 5, 1 to 4, 1 to 3, or 1 to 2. In some embodiments, p ranges from an average of 2 to 8, 2 to 7, 2 to 6, 2 to 5, 2 to 4 or 2 to 3. In other embodiments, p is an average of 1, 2, 3, 4, 5, 6, 7 or 8. In some embodiments, p ranges from an average of about 1 to about 20, about 1 to about 10, about 2 to about 10, about 2 to about 9, about 1 to about 8, about 1 to about 7, about 1 to about 6, about 1 to about 5, about 1 to about 4, about 1 to about 3, or about 1 to about 2. In some embodiments, p ranges from about 2 to about 8, about 2 to about 7, about 2 to about 6, about 2 to about 5, about 2 to about 4 or about 2 to about 3.

For example, when chemical activation of the protein results in formation of free thiol groups, the protein may be conjugated with a sulfhydryl reactive agent. In one aspect, the agent is one which is substantially specific for free thiol groups. Such agents include, for example, malemide, haloacetamides (e.g., iodo, bromo or chloro), haloesters (e.g., iodo, bromo or chloro), halomethyl ketones (e.g., iodo, bromo or chloro), benzylic halides (e.g., iodide, bromide or chloride), vinyl sulfone and pyridylthio.

The drug can be linked to the antibody or fragment by a linker. Suitable linkers include, for example, cleavable and non-cleavable linkers. A cleavable linker is typically susceptible to cleavage under intracellular conditions. Suitable cleavable linkers include, for example, a peptide linker cleavable by an intracellular protease, such as lysosomal protease or an endosomal protease. In exemplary embodiments, the linker can be a dipeptide linker, such as a valine-citrulline (val-cit), a phenylalanine-lysine (phe-lys) linker, or maleimidocapronic-valine-citruline-p-aminobenzyloxycarbonyl (mc-Val-Cit-PABA) linker. Another linker is Sulfosuccinimidyl-4-[N-maleimidomethyl]cyclohexane-1-carboxylate (smcc). Sulfo-smcc conjugation occurs via a maleimide group which reacts with sulfhydryls (thiols, -SH), while its Sulfo-NHS ester is reactive toward primary amines (as found in Lysine and the protein or peptide N-terminus). Yet another linker is maleimidocaproyl (mc). Other suitable linkers include linkers hydrolyzable at a specific pH or a pH range, such as a hydrazone linker. Additional suitable cleavable linkers include disulfide linkers. The linker may be covalently bound to the antibody to such an extent that the antibody must be degraded intracellularly in order for the drug to be released e.g. the mc linker and the like.

A linker can include a group for linkage to the antibody. For example, linker can include an amino, hydroxyl, carboxyl or sulfhydryl reactive groups (e.g., malemide, haloacetamides (e.g., iodo, bromo or chloro), haloesters (e.g., iodo, bromo or chloro), halomethyl ketones (e.g., iodo, bromo or chloro), benzylic halides (e.g., iodide, bromide or chloride), vinyl sulfone and pyridylthio).

In some embodiments, the drug moiety is a cytotoxic or cytostatic agent, an immunosuppressive agent, a radioisotope, a toxin, or the like. The conjugate can be used for inhibiting the multiplication of a tumor cell or cancer cell, causing apoptosis in a tumor or cancer cell, or for treating cancer in a patient. The conjugate can be used accordingly in a variety of settings for the treatment of animal cancers. The conjugate can be used to deliver a drug to a tumor cell or cancer cell. Without being bound by theory, in some embodiments, the conjugate binds to or associates with a cancer cell expressing GPC3, and the conjugate and/or drug can be taken up inside a tumor cell or cancer cell through receptor-mediated endocytosis.

Once inside the cell, one or more specific peptide sequences within the conjugate (e.g., in a linker) are hydrolytically cleaved by one or more tumor-cell or cancer-cell-associated proteases, resulting in release of the drug. The released drug is then free to migrate within the cell and induce cytotoxic or cytostatic or other activities. In some embodiments, the drug is cleaved from the antibody outside the tumor cell or cancer cell, and the drug subsequently penetrates the cell, or acts at the cell surface.

Examples of drug moieties or payloads are selected from the group consisting of DM1 (maytansine, N2'-deacetyl-N2'-(3-mercapto-1-oxopropyl)- or N2'-deacetyl-N2'-(3-mercapto-1-oxopropyl)-maytansine), mc-MMAD (6-maleimidocaproyl-monomethylauristatin-D or N-methyl-L-valyl-N-[(1S,2R)-2-methoxy-4-[(2S)-2-[(1R,2R)-1-methoxy-2-methyl-3-oxo-3-[[(1S)-2-phenyl-1-(2-thiazolyl)ethyl]amino]propyl]-1-pyrrolidinyl]-1-[(1S)-1-methylpropyl]-4-oxobutyl]-N-methyl-(9Cl)-L-valinamide), mc-MMAF (maleimidocaproyl-monomethylauristatin F or N-[6-(2,5-dihydro-2,5-dioxo-1H-pyrrol-1-yl)-1-oxohexyl]-N-methyl-L-valyl-L-valyl-(3R,4S,5S)-3-methoxy-5-methyl-4-(methylamino)heptanoyl-(αR, βR,2S)-β-methoxy-α-methyl-2-pyrrolidinepropanoyl-L-phenylalanine) and mc-Val-Cit-PABA-MMAE (6-maleimidocaproyl-ValcCit-(p-aminobenzyloxycarbonyl)-monomethylauristatin E or N-[[[4-[[N-[6-(2,5-dihydro-2,5-dioxo-1H-pyrrol-1-yl)-1-oxohexyl]-L-valyl-NS-(aminocarbonyl)-L-ornithyl]amino]phenyl]methoxy]carbonyl]-N-meth yl-L-valyl-N-[(1S,2R)-4-[(2S)-2-[(1R,2R)-3-[[(1R,2S)-2-hydroxy-1-methyl-2-phenylethyl]amino]-1-methoxy-2-methyl-3-oxopropyl]-1-pyrrolidinyl]-2-methoxy-1-[(1S)-1-methylpropyl]-4-oxobutyl]-N-methyl-L-valinamide). DM1 is a derivative of the tubulin inhibitor maytansine while MMAD, MMAE, and MMAF are auristatin derivatives. In some embodiments, the drug moiety is selected from the group consisting of mc-MMAF and mc-Val-Cit-PABA-MMAE. In some embodiments, the drug moiety is a maytansinoid or an auristatin.

The antibodies or fragments may be conjugated or fused to a therapeutic agent, which may include detectable labels such as radioactive labels, an immunomodulator, a hormone, an enzyme, an oligonucleotide, a photoactive therapeutic or diagnostic agent, a cytotoxic agent, which may be a drug or a toxin, an ultrasound enhancing agent, a non-radioactive label, a combination thereof and other such agents known in the art.

The antibodies can be detectably labeled by coupling it to a chemiluminescent compound. The presence of the chemiluminescent-tagged antigen-binding polypeptide is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

The antibodies can also be detectably labeled using fluorescence emitting metals such as ¹⁵²Eu, or others of the lanthanide series. These metals can be attached to the antibody using such metal chelating groups as diethylenetriaminepentacetic acid (DTPA) or ethylenediaminetetraacetic acid (EDTA). Techniques for conjugating various moieties to an antibody are well known, *see, e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. (1985); Hellstrom et al., "Antibodies For Drug Delivery", in Controlled Drug Delivery (2nd Ed.), Robinson et al., (eds.), Marcel Dekker, Inc., pp. 623-53 (1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), Academic Press pp. 303-16 (1985), and Thorpe et al., "The Preparation And Cytotoxic Properties Of Antibody-Toxin Conjugates ", Immunol. Rev. (52:119-58 (1982)).

### Multi-functional Molecules

As a molecule preferentially expressed on tumor cells, an antibody or antigen-binding fragment specific to GPC3 can be combined with a second or more fragment specific to an immune cell to generate a bispecific antibody or multispecific antibody.

In some embodiments, the immune cell is selected from the group consisting of a T cell, a B cell, a monocyte, a macrophage, a neutrophil, a dendritic cell, a phagocyte, a natural killer cell, an eosinophil, a basophil, and a mast cell.

In some embodiment, the second specificity is to CD3, CD47, PD1, PD-L1, LAG3, TIM3, CTLA4, VISTA, CSFR1, A2AR, CD73, CD39, CD40, CEA, HER2, CMET, 4-1BB, OX40, SIRPA CD16, CD28, ICOS, CTLA4, BTLA, TIGIT, HVEM, CD27, VEGFR, or VEGF.

Different formats of bispecific antibodies are also provided. In some embodiments, each of the anti-GPC3 fragment and the second fragment each is independently selected from a Fab fragment, a single-chain variable fragment (scFv), or a single-domain antibody. In some embodiments, the bispecific antibody further includes a Fc fragment.

Bifunctional molecules that include not just antibody or antigen binding fragment are also provided. As a tumor antigen targeting molecule, an antibody or antigen-binding fragment specific to GPC3, such as those described here, can be combined with an immune cytokine or ligand optionally through a peptide linker. The linked immune cytokines or ligands include, but not limited to, IL-2, IL-3, IL-4, IL-5, IL-6, IL-7, IL-10, IL-12, IL-13, IL-15, GM-CSF, TNF-α, CD40L, OX40L, CD27L, CD30L, 4-1BBL, LIGHT and GITRL. Such bifunctional molecules can combine the immune checkpoint blocking effect with tumor site local immune modulation.

### Chimeric Antigen Receptors, Polynucleotides, and Methods of Preparing the Antibodies

Also provided, in one embodiment, is a chimeric antigen receptor (CAR) that includes the antibody or fragment thereof of the present disclosure as a targeting unit. In some embodiments, the CAR includes an antibody or fragment thereof of the present disclosure, a transmembrane domain, a costimulatory domain, and a CD3ξ intracellular domain.

A transmembrane domain can be designed to be fused to the extracellular domain which includes the antibody or fragment, optionally through a hinge domain. It can similarly be fused to an intracellular domain, such as a costimulatory domain. In some embodiments, the transmembrane domain can include the natural transmembrane region of a costimulatory domain (e.g., the TM region of a CD28T or 4- IBB employed as a costimulatory domain) or the natural transmembrane domain of a hinge region (e.g., the TM region of a CD8 alpha or CD28T employed as a hinge domain).

In some embodiments, the transmembrane domain can include a sequence that spans a cell membrane, but extends into the cytoplasm of a cell and/or into the extracellular space. For example, a transmembrane can include a membrane-spanning sequence which itself can further include 1, 2, 3, 4, 5, 6, 7, 8, 9, 10 or more amino acids that extend into the cytoplasm of a cell, and/or the extracellular space. Thus, a transmembrane domain includes a membrane-spanning region, yet can further comprise an amino acid(s) that extend beyond the internal or external surface of the membrane itself; such sequences can still be considered to be a "transmembrane domain."

In some embodiments, the transmembrane domain is fused to the cytoplasmic domain through a short linker. Optionally, the short peptide or polypeptide linker, preferably between 2 and 10 amino acids in length can form the linkage between the transmembrane domain and a proximal cytoplasmic signaling domain of the chimeric receptor. A glycine-serine doublet (GS), glycine-serine-glycine triplet (GSG), or alanine- alanine-alanine triplet (AAA) provides a suitable linker.

In some embodiments, the CAR further includes a costimulatory domain. In some embodiments, the costimulatory domain is positioned between the transmembrane domain and an activating domain. Example costimulatory domains include, but are not limited to, CD2, CD3 delta, CD3 epsilon, CD3 gamma, CD4, CD7, CD8a, CD8 , CD11a (ITGAL), CD11b (ITGAM), CD11c (ITGAX), cD11d (ITGAD), CD18 (ITGB2), CD19 (B4), CD27 (T FRSF7), CD28, CD28T, CD29 (ITGB1), CD30 (TNFRSF8), CD40 (TNFRSF5), CD48 (SLAMF2), CD49a (ITGA1), CD49d (ITGA4), CD49f (ITGA6), CD66a (CEACAM1), CD66b (CEACAM8), CD66c (CEACAM6), CD66d (CEACAM3), CD66e (CEACAM5), CD69 (CLEC2), CD79A (B-cell antigen receptor complex-associated alpha chain), CD79B (B-cell antigen receptor complex- associated beta chain), CD84 (SLAMF5), CD96 (Tactile), CD 100 (SEMA4D), CD 103 (ITGAE), CD134 (OX40), CD137 (4-1BB), CD150 (SLAMF1), CD158A (KIR2DL1), CD158B1 (KIR2DL2), CD158B2 (KIR2DL3), CD158C (KIR3DP1), CD158D (KIRDL4), CD158F1 (KIR2DL5A), CD158F2 (KIR2DL5B), CD158K (KTR3DL2), CD160 (BY55), CD162 (SELPLG), CD226 (DNAM1), CD229 (SLAMF3), CD244 (SLAMF4), CD247 (CD3-zeta), CD258 (LIGHT), CD268 (BAFFR), CD270 (T FSF14), CD272 (BTLA), CD276 (B7-H3), CD279 (PD-1), CD314 (KG2D), CD319 (SLAMF7), CD335 (K-p46), CD336 (K-p44), CD337 (K-p30), CD352 (SLAMF6), CD353 (SLAMF8), CD355 (CRTAM), CD357 (TNFRSF 18), inducible T cell co-stimulator (ICOS), LFA-1 (CD11a/CD18), KG2C, DAP- 10, ICAM-1, Kp80 (KLRF1), IL-2R beta, IL-2R gamma, IL-7R alpha, LFA-1, SLAMF9, LAT, GADS (GrpL), SLP-76 (LCP2), PAG1/CBP, a CD83 ligand, Fc gamma receptor, MHC class 1 molecule, MHC class 2 molecule, a TNF receptor protein, an immunoglobulin protein, a cytokine receptor, an integrin, activating NK cell receptors, a Toll ligand receptor, and fragments or combinations thereof.

In some embodiments, the cytoplasmic portion of the CAR also includes a signaling/activation domain. In one embodiment, the signaling/activation domain is the CD3ξ domain, or is an amino acid sequence having at least about 80%, 85%, 90%, 95%, 98% or 99% sequence identity to the CD3ξ domain.

The present disclosure also provides isolated polynucleotides or nucleic acid molecules encoding the antibodies, variants or derivatives thereof of the disclosure, or the CAR. The polynucleotides of the present disclosure may encode the entire heavy and light chain variable regions of the antigen-binding polypeptides, variants or derivatives thereof on the same polynucleotide molecule or on separate polynucleotide molecules. Additionally, the polynucleotides of the present disclosure may encode portions of the heavy and light chain variable regions of the antigen-binding polypeptides, variants or derivatives thereof on the same polynucleotide molecule or on separate polynucleotide molecules.

Methods of making antibodies are well known in the art and described herein. In certain embodiments, both the variable and constant regions of the antigen-binding polypeptides of the present disclosure are fully human. Fully human antibodies can be made using techniques described in the art and as described herein. For example, fully human antibodies against a specific antigen can be prepared by administering the antigen to a transgenic animal which has been modified to produce such antibodies in response to antigenic challenge, but whose endogenous loci have been disabled. Exemplary techniques that can be used to make such antibodies are described in U.S. patents: 6,150,584; 6,458,592; 6,420,140 which are incorporated by reference in their entireties.

### Treatment and Uses

As described herein, the antibodies, variants, derivatives or antibody-drug conjugates of the present disclosure may be used in certain treatment and diagnostic methods.

The present disclosure is further directed to antibody-based therapies which involve administering the antibodies, fragments, or antibody-drug conjugates of the disclosure to a patient such as an animal, a mammal, and a human for treating one or more of the disorders or conditions described herein. Therapeutic compounds of the disclosure include, but are not limited to, antibodies of the disclosure (including variants and derivatives thereof as described herein) and nucleic acids or polynucleotides encoding antibodies of the disclosure (including variants and derivatives thereof as described herein).

The antibodies of the disclosure can also be used to treat or inhibit cancer. As provided above, GPC3 can be overexpressed in tumor cells, in particular liver, gastric, pancreatic, esophageal, ovarian, and lung tumors. Inhibition of GPC3 has been shown to be useful for treating the tumors.

Accordingly, in some embodiments, provided are methods for treating a cancer in a patient in need thereof. The method, in one embodiment, entails administering to the patient an effective amount of an antibody, fragment, or antibody-drug conjugate of the present disclosure. In some embodiments, at least one of the cancer cells (e.g., stromal cells) in the patient over-express GPC3.

Cellular therapies, such as chimeric antigen receptor (CAR) T-cell therapies, are also provided in the present disclosure. A suitable cell can be used, that is transduced with a vector that encodes, or put in contact with, an CAR that includes an anti-GPC3 antibody of the present disclosure (or alternatively engineered to express an anti-GPC3 antibody of the present disclosure). Upon such contact or engineering, the cell can then be introduced to a cancer patient in need of a treatment. The cancer patient may have a cancer of any of the types as disclosed herein. The cell (e.g., T cell) can be, for instance, a tumor-infiltrating T lymphocyte, a CD4+ T cell, a CD8+ T cell, or the combination thereof, without limitation.

In some embodiments, the cell was isolated from the cancer patient him- or her-self. In some embodiments, the cell was provided by a donor or from a cell bank. When the cell is isolated from the cancer patient, undesired immune reactions can be minimized.

Non-limiting examples of cancers include bladder cancer, breast cancer, colorectal cancer, endometrial cancer, esophageal cancer, head and neck cancer, kidney cancer, leukemia, liver cancer, lung cancer, lymphoma, melanoma, pancreatic cancer, prostate cancer, and thyroid cancer. In some embodiments, the cancer is one or more of gastric, pancreatic, esophageal, ovarian, and lung cancers.

Additional diseases or conditions associated with increased cell survival, that may be treated, prevented, diagnosed and/or prognosed with the antibodies or variants, or derivatives thereof of the disclosure include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyo sarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma and retinoblastoma.

A specific dosage and treatment regimen for any particular patient will depend upon a variety of factors, including the particular antibodies, variant or derivative thereof used, the patient's age, body weight, general health, sex, and diet, and the time of administration, rate of excretion, drug combination, and the severity of the particular disease being treated. Judgment of such factors by medical caregivers is within the ordinary skill in the art. The amount will also depend on the individual patient to be treated, the route of administration, the type of formulation, the characteristics of the compound used, the severity of the disease, and the desired effect. The amount used can be determined by pharmacological and pharmacokinetic principles well known in the art.

Methods of administration of the antibody, fragment, or antibody-drug conjugate or include but are not limited to intradermal, intramuscular, intraperitoneal, intravenous, subcutaneous, intranasal, epidural, and oral routes. The antigen-binding polypeptides or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Thus, pharmaceutical compositions containing the antigen-binding polypeptides of the disclosure may be administered orally, rectally, parenterally, intracistemally, intravaginally, intraperitoneally, topically (as by powders, ointments, drops or transdermal patch), bucally, or as an oral or nasal spray.

The term "parenteral" as used herein refers to modes of administration which include intravenous, intramuscular, intraperitoneal, intrasternal, subcutaneous and intra-articular injection and infusion.

Administration can be systemic or local. In addition, it may be desirable to introduce the antibodies of the disclosure into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

It may be desirable to administer the antigen-binding polypeptides or compositions of the disclosure locally to the area in need of treatment; this may be achieved by, for example, and not by way of limitation, local infusion during surgery, topical application, e.g., in conjunction, with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the disclosure, care must be taken to use materials to which the protein does not absorb.

The amount of the antibodies, fragments, or antibody-drug conjugates of the disclosure which will be effective in the treatment, inhibition and prevention of an inflammatory, immune or malignant disease, disorder or condition can be determined by standard clinical techniques. In addition, *in vitro* assays may optionally be employed to help identify optimal dosage ranges. The precise dose to be employed in the formulation will also depend on the route of administration, and the seriousness of the disease, disorder or condition, and should be decided according to the judgment of the practitioner and each patient's circumstances. Effective doses may be extrapolated from dose-response curves derived from *in vitro* or animal model test systems.

As a general proposition, the dosage administered to a patient of the antibodies, fragments, or antibody-drug conjugates of the present disclosure is typically 0.001 mg/kg to 100 mg/kg of the patient's body weight, between 0.01 mg/kg and 20 mg/kg of the patient's body weight, or 0.5 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the disclosure may be reduced by enhancing uptake and tissue penetration (e.g., into the brain) of the antibodies by modifications such as, for example, lipidation.

In an additional embodiment, the compositions of the disclosure are administered in combination with cytokines. Cytokines that may be administered with the compositions of the disclosure include, but are not limited to, IL-2, IL-3, IL-4, IL-5, IL-6, IL-7, IL-10, IL-12, IL-13, IL-15, anti-CD40, CD40L, and TNF-α.

In additional embodiments, the compositions of the disclosure are administered in combination with other therapeutic or prophylactic regimens, such as, for example, radiation therapy.

### Compositions

The present disclosure also provides pharmaceutical compositions. Such compositions comprise an effective amount of an antibody, fragment, or antibody-drug conjugate, and an acceptable carrier. In some embodiments, the composition further includes a second anticancer agent (e.g., an immune checkpoint inhibitor).

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. Further, a "pharmaceutically acceptable carrier" will generally be a non-toxic solid, semisolid or liquid filler, diluent, encapsulating material or formulation auxiliary of any type.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents such as acetates, citrates or phosphates. Antibacterial agents such as benzyl alcohol or methyl parabens; antioxidants such as ascorbic acid or sodium bisulfite; chelating agents such as ethylenediaminetetraacetic acid; and agents for the adjustment of tonicity such as sodium chloride or dextrose are also envisioned. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in Remington's Pharmaceutical Sciences by E. W. Martin, incorporated herein by reference. Such compositions will contain a therapeutically effective amount of the antigen-binding polypeptide, preferably in purified form, together with a suitable amount of carrier so as to provide the form for proper administration to the patient. The formulation should suit the mode of administration. The parental preparation can be enclosed in ampoules, disposable syringes or multiple dose vials made of glass or plastic.

In an embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the disclosure can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

### EXAMPLES

### Example 1: Generation of Mouse Monoclonal Antibodies against Human GPC3

This example describes the generation of anti-human-GPC3 mouse monoclonal antibodies using the hybridoma technology.

*Antigen*: human GPC3-His protein.

*Immunization:* To generate mouse monoclonal antibodies targeting human GPC3, Balb/c mice and C57BL/6 mice were first immunized with the GPC3-His protein. The immunized mice were subsequently boosted with the GPC3-His protein. To select mice producing antibodies that bound to GPC3 protein, the serum of immunized mice was subjected to the antibody titer evaluation by ELISA. Briefly, microtiter plates were coated with human GPC3 protein at 0.5 µg/ml in ELISA coating buffer, 100 µl/well at 4°C overnight, then blocked with 150 µl/well of 1% BSA. Dilutions of serum from immunized mice were added to each well and incubated for 1-2 hours at 37°C. The plates were washed with PBS/Tween and then incubate with anti-mouse IgG antibody conjugated with Horse Radish Peroxidase (HRP) for 1 hour at 37°C. After washing, the plates were developed with TMB substrate and analyzed by spectrophotometer at OD 450nm. After a few rounds of immunization, immune responses were also tested by serum FACS against GPC3-CHOK1 cell line with CHOK1 parental cell line served as negative control. The resulting mice were used for fusions. The hybridoma supernatants were screened by ELISA.

*Cell fusion:* Fusion was performed by electro fusion. Fused cells were plated into 50 96-well plates for each fusion.

*Screening:* The supernatants were screened by ELISA against recombinant human (rh) GPC3-His protein and counter screening antigen. Then, positive supernatants underwent confirmative screening with cell based binding against GPC3-CHOK1 cell line.

*Subcloning and screening:* positive primary clones from each fusion were subcloned by limiting dilution to ensure that the subclones were derived from a single parental cell. Subcloning were screened in the same approach as primary clones and culture supernatant of positive clones underwent additional confirmative screening by affinity ranking.

Hybridoma clones 18C1D9, 52H5D3B8, 163E7D12, 172B4E9, 152E9F7 and 171F9C5 were selected for further analysis. The amino acid sequences of the variable regions of 18C1D9, 52H5D3B8, 163E7D12, 172B4E9, 152E9F7 and 171F9C5 are listed in Table 1 below.

**Table 1. Sequences of the variable regions of 18C1D9, 52H5D3B8, 163E7D12, 172B4E9, 152E9F7 and 171F9C5**

| **Name** | **Sequence** | **SEQ ID NO:** |
|---|---|---|
| 18C1D9 VH | QVQLQQSGAELVRPGASVTLSCKASGYTFSDYEMHWVKQTPVHGLEWIGA IDPETGGTAYNQKFKGKATLTADKSSSTAYMELRSLTSEDSAVYYCTRYY SYAYWGQGTLVTVSA | 1 |
| 18C1D9 VL | DVVMTQSPLSLPVSLGDQASISCRSRQSLVHSNGNTYLQWYLQKPGQSPK LLIYKVSNRFSGVPDRFSGSGSGTDFTLKISRVEAEDLGVYFCFQSIHVP YTFGGGTKLEKK | 2 |
| 52H5D3B8 VH | QVQLQQSGAELVRPGASVKLSCKALGYTFTDYEMHWVKQTPVHGLEWIGA IHPGSGGTAYNQKFKGKATLTADKFSSTGYMELSSLTSEDSAVYYCTRFY SYAYWGQGTLVTVSA | 3 |
| 52H5D3B8 VL | DVVMTQTPLSLPVSLGDQASISCRSSQSLVHSNGNTYLQWYLQKPGQSPK LLIYKVSNRFSGVPDRFSGSGSGTDFILKISRVEAEDLGVYFCSQSIHVP YTFGGGTKLEIK | 4 |
| 163E7D12 VH | QVQLQQSGAELVRPGASVTLSCKASGYTFTDYEMHWVKQTPVHGLEWIGA IGPETGSTAYNQRFKGKAIVTADKSSSTAYMELRSLTSEDSAVYYCTRYY SFAYWGQGTLVTVSA | 5 |
| 163E7D12 VL | DVVMTQTPLSLPVSLGGQASISCRSSQSLVHSNGNTYLQWYLQKPGQSPK LLIFKVSNRFSGVPDRFSGSGSGTDFTLKISRVEAEDLGVYFCFQSIHVP YTFGGGTKLEIK | 6 |
| 172B4E9 VH | QVQLQQSGAELVRPGASVTLSCKASGYTFTDYEIHWVKQTPVHGLEWIGA IDPATGDTAYTQKFKGKAILTADTSSNTVYMELRGLTSEDSAVHFCTRFY SYAYWGQGTLVTVSA | 7 |
| 172B4E9 VL | DVVMTQTPLSLPVSLGDQASISCRSSQSLVHTNGHTYLQWYLQKSGQSPR LLIFKVSNRFSGVPDRFSGSGSGTDFKLKISRVEAEDLGVYFCSQSTHVP YTFGGGTKLEIK | 8 |
| 152E9F7 VH | QVQLQQSGAELVRPGASVTLSCKASGYTFTDYEIHWVKQTPVHGLEWIGA IDPETGDTAYTQKFKGKAILTADKSSNTVYMELRSLTSEDSAVYYCTRFY SYAYWGQGTLVTVSA | 9 |
| 152E9F7 VL | DVVMTQTPLSLPVSLGDQASISCRSSQSLVHTNGHTYLQWYLQKSGQSPK LLIYKVSNRFSGVPDRFSGSGSGTDFTLKISRVEAEDLGVYFCSQSTHVP YTFGGGTKLEIK | 10 |
| 171F9C5 VH | QVQLQQSGAELVRPGASVTLSCKASGYTFTDYEIHWVKQTPVHGLEWIGA IDPETDNTAYSQKFKGKAILTADKSSSTAYMELRSLTSEDSAVYYCTRYY SYAYWGQGTLVTVSA | 11 |
| 171F9C5 VL | DVVMTQTPLSLPVSLGDQASISCRSSQSPVHRNGNTYLHWYLQKPGQSPK LLIFKVSNRFSGVPDRFSGSGSGTDFTLKISRVAAEDLGVYFCSQTTHVP YTFGGGTKLEIK | 12 |

**Table 1A. CDR sequences of 18C1D9**

| **18C1D9** | **Sequence** | **SEQ ID NO:** |
|---|---|---|
| CDRH1 | DYEMH | 13 |
| CDRH2 | AIDPETGGTAYNQKFKG | 14 |
| CDRH3 | YYSYAY | 15 |
| CDRL1 | RSRQSLVHSNGNTYLQ | 16 |
| CDRL2 | KVSNRFS | 17 |
| CDRL3 | FQSIHVPYT | 18 |

**Table 7β. CDR sequences of 52H5D3B8**

| **52H5D3B8** | **Sequence** | **SEQ ID NO:** |
|---|---|---|
| CDRH1 | DYEMH | 13 |
| CDRH2 | AIHPGSGGTAYNQKFKG | 19 |
| CDRH3 | FYSYAY | 20 |
| CDRL1 | RSSQSLVHSNGNTYLQ | 21 |
| CDRL2 | KVSNRFS | 17 |
| CDRL3 | SQSIHVPYT | 22 |

**Table 1C. CDR sequences of 163E7D12**

| **163E7D12** | **Sequence** | **SEQ ID NO:** |
|---|---|---|
| CDRH1 | DYEMH | 13 |
| CDRH2 | AIGPETGSTAYNQRFKG | 23 |
| CDRH3 | YYSFAY | 24 |
| CDRL1 | RSSQSLVHSNGNTYLQ | 21 |
| CDRL2 | KVSNRFS | 17 |
| CDRL3 | FQSIHVPYT | 18 |

**Table ID. CDR sequences of 172B4E9**

| **172B4E9** | **Sequence** | **SEQ ID NO:** |
|---|---|---|
| CDRH1 | DYEIH | 25 |
| CDRH2 | AIDPATGDTAYTQKFKG | 26 |
| CDRH3 | FYSYAY | 20 |
| CDRL1 | RSSQSLVHTNGHTYLQ | 27 |
| CDRL2 | KVSNRFS | 17 |
| CDRL3 | SQSTHVPYT | 28 |

**Table IE. CDR sequences of 152E9F7**

| **152E9F7** | **Sequence** | **SEQ ID NO:** |
|---|---|---|
| CDRH1 | DYEIH | 25 |
| CDRH2 | AIDPETGDTAYTQKFKG | 29 |
| CDRH3 | FYSYAY | 20 |
| CDRL1 | RSSQSLVHTNGHTYLQ | 27 |
| CDRL2 | KVSNRFS | 17 |
| CDRL3 | SQSTHVPYT | 28 |

**Table IF. CDR sequences of 171F9C5**

| **171F9C5** | **Sequence** | **SEQ ID NO:** |
|---|---|---|
| CDRH1 | DYEIH | 25 |
| CDRH2 | AIDPETDNTAYSQKFKG | 30 |
| CDRH3 | YYSYAY | 15 |
| CDRL1 | RSSQSPVHRNGNTYLH | 31 |
| CDRL2 | KVSNRFS | 17 |
| CDRL3 | SQTTHVPYT | 32 |

### Example 2: Binding Affinity of Mouse and Chimeric mAbs

### Affinity ranking of mouse monoclonal antibodies

The binding of mouse monoclonal antibodies to human GPC3-his was tested with Biacore using a capture method. The antibodies were captured using CM5 sensor chip. A single dose of human GPC3-his tag protein was injected over captured antibody for 2 mins at a flow rate of 30 µl/min. The antigen was allowed to dissociate for 360s. All the experiments were carried out on a Biacore 8K. Data analysis was carried out using Biacore 8K evaluation software.

As shown in the results of **Table 2A,** all mouse monoclonal antibodies exhibited high binding affinity to the recombinant GPC3 protein.

**Table 2A. Affinity ranking by Biacore**

| **Abs** | **GPC3-His** | | |
|---|---|---|---|
| | **ka (1/Ms)** | **kd (1/s)** | **KD (M)** |
| **18C1D9** | 2.62E+05 | 4.12E-04 | **1.57E-09** |
| **52H5D3B8** | 2.28E+05 | 3.17E-04 | **1.39E-09** |
| **163E7D12** | 2.23E+05 | 1.69E-04 | **7.55E-10** |
| **172B4E9** | 2.02E+05 | 9.01E-05 | **4.46E-10** |
| **152E9F7** | 1.84E+05 | 1.62E-04 | **8.81E-10** |
| **171F9C5** | 1.62E+05 | 1.99E-04 | **1.22E-09** |

The variable region of mouse antibodies 18C1D9, 52H5D3B8, 163E7D12, 172B4E9, 152E9F7 and 171F9C5 were then fused to human IgG1 to generate chimeric mAbs.

### Full kinetic affinity of 52H5D3B8 by Biacore

The binding of the chimeric 52H5D3B8 mAb to human GPC3-his was tested with Biacore using a capture method. The 52H5D3B8 mAb was captured using CM5 sensor chip. A serial dilution of human GPC3-his tag protein was injected over captured antibody for 2 mins at a flow rate of 30 µl/min. The antigen was allowed to dissociate for 360s. All the experiments were carried out on a Biacore 8K. Data analysis was carried out using Biacore 8K evaluation software.

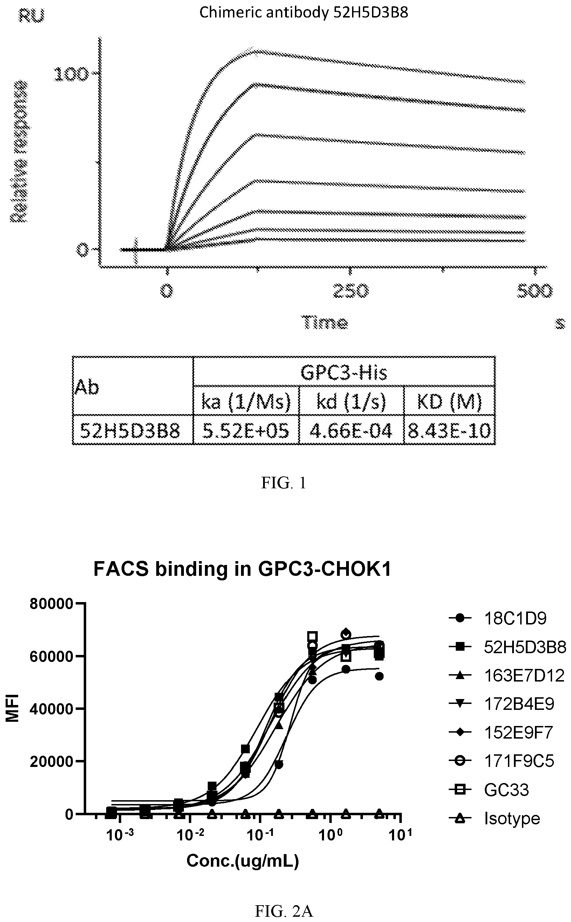

As shown in the results of **FIG.** 1 and **Table 2B** below, the 52H5D3B8 antibody exhibited high binding affinity to the recombinant GPC3 protein.

**Table 2B. Affinity measured by Biacore**

| **Abs** | **GPC3-His** | | |
|---|---|---|---|
| | **ka (1/Ms)** | **kd (1/s)** | **KD (M)** |
| **52H5D3B8** | 5.52 E+5 | 4.66E-4 | **8.43E-10** |

### Example 3: The Binding Activity to GPC3 Antigen

### FACS testing of Chimeric antibodies

Cell-based binding: FACS was used to evaluate the binding activity of chimeric mAbs (18C1D9, 52H5D3B8, 163E7D12, 172B4E9, 152E9F7 and 171F9C5) with comparison to a known GPC3 antibody GC33 (Chugai Pharmaceutical) on human GPC3 over-expressed CHOK1 cells.

Briefly, GPC3-CHOK1 cells were washed by FACS buffer and divided to each well with 3-fold serially diluted chimeric mAbs or GC33 mAb starting at 5 µg/mL at 4°C for 40 mins. After washing by FACS buffer, Alexa Fluor^{®} 647 AffiniPure Goat Anti-Human IgG (H+L) was added to each well and incubated at 4°C for 30 mins. Samples were washed twice with FACS buffer. The mean florescence intensity (MFI) of Alexa Fluor^{®} 647 was evaluated. The EC50 of each mAbs on GPC3-CHOK1 cells is listed in **Table 3A** below. 52H5D3B8 chimeric antibody showed higher cell-based binding efficiency than GC33 **(****FIG. 2A** and **Table 3A).**

**Table 3A. The binding activity to GPC3-CHOKI cells**

| **EC50 mAbs** | **GPC3-CHOK1 cells** |
|---|---|
| **18C1D9** | 0.2383 µg/mL |
| **52H5D3B8** | 0.0909 µg/mL |
| **163E7D12** | 0.1701 µg/mL |
| **172B4E9** | 0.2686 µg/mL |
| **152E9F7** | 0.1395 µg/mL |
| **171F9C5** | 0.1419 µg/mL |
| **GC33** | 0.1222 µg/mL |

### FACS testing of 52H5D3B8 chimeric antibody

Cell-based binding: FACS was used to evaluate the binding activity of 52H5D3B8 chimeric mAbs with comparison to a known GPC3 antibody GC33 (Chugai Pharmaceutical) on human GPC3 over-expressed CHOK1 cells, HEPG2 cells, Hep3B cells and Huh-7 cells.

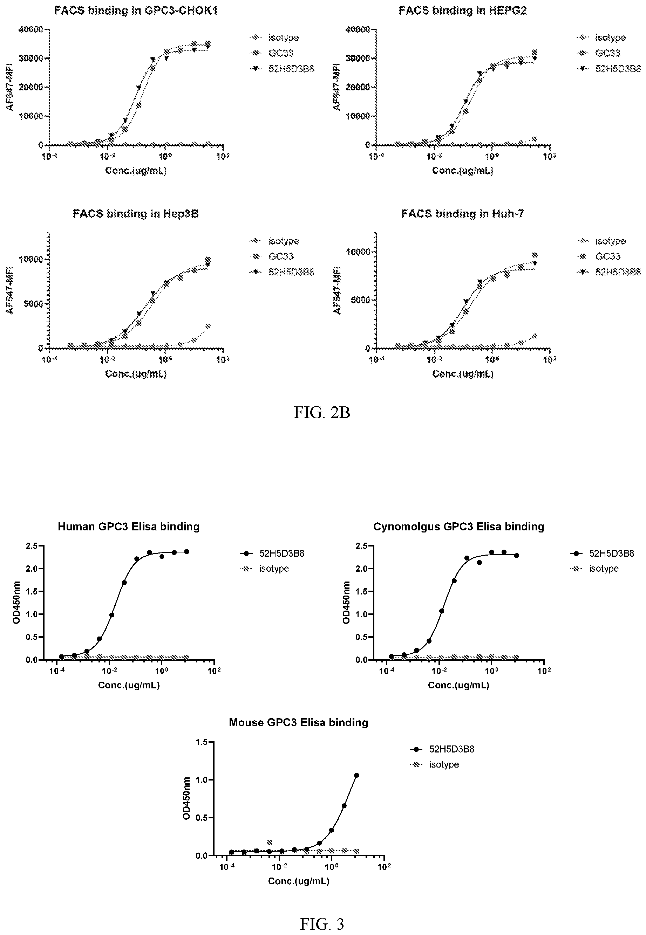

Briefly, GPC3-CHOK1 cells, HEPG2 cells, Hep3B cells or Huh-7 cells were firstly incubated with Fc blocking reagent at 4°C for 15 mins. Cells were washed by FACS buffer and divided to each well with 3-fold serially diluted 52H5D3B8 chimeric mAbs or GC33 mAb starting at 30 µg/mL at 4°C for 60 mins. Cells were washed by FACS buffer and fixed with 2% PFA at RT for 15 mins. After washing by FACS buffer, Alexa Fluor^{®} 647 AffiniPure Goat Anti-Human IgG was added to each well and incubated at RT for 30 mins. Samples were washed twice with FACS buffer. The mean florescence intensity (MFI) of Alexa Fluor^{®} 647 was evaluated by MACS Quant Analyzer 16. As shown in **FIG. 2B**, 52H5D3B8 bound to GPC3-CHOK1 cells, HEPG2 cells, Hep3B cells or Huh-7 cells with comparable binding efficiency, which is higher than GC33. The EC50 of each mAbs on GPC3-CHOK1 cells, HEPG2 cells, Hep3B cells and Huh-7 cells is listed in **Table 3B** below. 52H5D3B8 antibody showed higher cell-based binding efficiency than GC33 **(****FIG. 2B** and **Table 3B).**

**Table 3B. The binding activity to GPC3-CHOK1 cells, HEPG2 cells, Hep3B cells and Huh-7 cells**

| **EC50 mAbs** | **GPC3-CHOK1 cells** | **HEPG2 cells** | **Hep3B cells** | **Huh-7 cells** |
|---|---|---|---|---|
| **52H5D3B8** | 0.0922 µg/mL | 0.1111 µg/mL | 0.1915 µg/mL | 0.0986 µg/mL |
| **GC33** | 0.1656 µg/mL | 0.1808 µg/mL | 0.3411 ug/mL | 0.1816 ug/mL |

### Cross species activity

ELISA testing was carried out to evaluate the binding of chimeric antibodies to human, mouse and cynomolgus GPC3, respectively.

Briefly, microtiter plates were coated with human, mouse and cynomolgus GPC3 proteins at 1 µg/ml in PBS, 100 µl/well at 4°C overnight, then blocked with 150 µl/well of 1% BSA. Three-fold dilutions of chimeric antibodies starting from 9 µg/ml were added to each well and incubated for 1 hour at 37 °C. The plates were washed with PBS/Tween and then incubate with mouse-anti-human IgG Fc antibody conjugated with Horse Radish Peroxidase (HRP) for 30 mins at 37 °C. After washing, the plates were developed with TMB substrate and analyzed by spectrophotometer at OD 450nm. The 52H5D3B8 antibody bound to human, cynomolgus GPC3 and mouse GPC3 **(****FIG. 3** and **Table 4).**

**Table 4A. Cross species activity of 52H5D3B8 to Human GPC3**

| | **Human** |
|---|---|
| EC50 of52H5D3B8 | 17.03ng/ml |

**Table 4B. Cross species activity of 52H5D3B8 to Cynomolgus and mouse GPC3**

| | **Cynomolgus** | **Mouse** |
|---|---|---|
| EC50 of52H5D3B8 | 15.25ng/ml | 4948ng/ml |

### Example 4: Optimization of 52H5D3B8 Chimeric mAbs

This example describes the optimization of 52H5D3B8 chimeric mAb by mutation of CDR3s to enhance the binding activity of 52H5D3B8 chimeric mAb on human GPC3.

Mutations: As the CDR3 domains of antibody play a crucial role in interacting with antigen, mutation has been made on the CDR3 domain of 52H5D3B8 chimeric mAb. Both CDRH3 and CDRL3 has been designed to have two candidate mutation sites, respectively. Together, 15 candidate combinations of CDRH3 and CDRL3 sequences are listed in **Table 5** below.

**Table 5. Sequences of the variable regions of mutants of 52H5D3B8**

| **Name** | **Sequence** | **SEQ ID NO:** |
|---|---|---|
| 52H5D3B8 VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSYAYWG QGTLVTVSA | 3 |
| 52H5D3B8 VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSIHVPYTFGGG TKLEIK | 4 |
| 52H5D3B8-1# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSYAYWG QGTLVTVSA | 35 |
| 52H5D3B8-1# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSIHVPYTFGGG TKLEIK | 4 |
| 52H5D3B8-2# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSFAYWG QGTLVTVSA | 67 |
| 52H5D3B8-2# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSIHVPYTFGGG TKLEIK | 4 |
| 52H5D3B8-3# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSYAYWG QGTLVTVSA | 3 |
| 52H5D3B8-3# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSIHVPYTFGGG TKLEIK | 40 |
| 52H5D3B8-4# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSYAYWG QGTLVTVSA | 3 |
| 52H5D3B8-4# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSTHVPYTFGGG TKLEIK | 68 |
| 52H5D3B8-5# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSFAYWG QGTLVTVSA | 69 |
| 52H5D3B8-5# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSIHVPYTFGGG TKLEIK | 4 |
| 52H5D3B8-6# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSYAYWG QGTLVTVSA | 35 |
| 52H5D3B8-6# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSIHVPYTFGGG TKLEIK | 40 |
| 52H5D3B8-7# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSYAYWG QGTLVTVSA | 35 |
| 52H5D3B8-7# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSTHVPYTFGGG TKLEIK | 68 |
| 52H5D3B8-8# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSFAYWG QGTLVTVSA | 67 |
| 52H5D3B8-8# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSIHVPYTFGGG TKLEIK | 40 |
| 52H5D3B8-9# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSFAYWG QGTLVTVSA | 67 |
| 52H5D3B8-9# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSTHVPYTFGGG TKLEIK | 68 |
| 52H5D3B8-10# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSYAYWG QGTLVTVSA | 3 |
| 52H5D3B8-10# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSTHVPYTFGGG TKLEIK | 70 |
| 52H5D3B8-11# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRFYSFAYWG QGTLVTVSA | 67 |
| 52H5D3B8-11# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSTHVPYTFGGG TKLEIK | 70 |
| 52H5D3B8-12# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSYAYWG QGTLVTVSA | 35 |
| 52H5D3B8-12# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSTHVPYTFGGG TKLEIK | 70 |
| 52H5D3B8-13# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSFAYWG QGTLVTVSA | 69 |
| 52H5D3B8-13# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCSQSTHVPYTFGGG TKLEIK | 68 |
| 52H5D3B8-14# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSFAYWG QGTLVTVSA | 69 |
| 52H5D3B8-14# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSIHVPYTFGGG TKLEIK | 40 |
| 52H5D3B8-15# VH | QVQLQQSGAELVRPGASVKLSCKALGYTFTDYEMHWVKQTPVHGLEWIGAIHP GSGGTAYNQKFKGKATLTADKFSSTGYMELSSLTSEDSAVYYCTRYYSFAYWG QGTLVTVSA | 69 |
| 52H5D3B8-15# VL | DVVMTQTPLSLPVSLGDQASISCRSSQSLVHSNGNTYLQWYLQKPGQSPKLLI YKVSNRFSGVPDRFSGSGSGTDFILKISRVEAEDLGVYFCFQSTHVPYTFGGG TKLEIK | 70 |

**Table 5A. CDR sequences of 52H5D3B8-1#**

| **52H5D3B8-1#** | **Sequence** | **SEQ ID NO:** |
|---|---|---|
| CDRH3 | YYSYAY | 15 |
| CDRL3 | SQSIHVPYT | 22 |

**Table 5B. CDR sequences of 52H5D3B8-2#**

| **52H5D3B8-2#** | **Sequence** | **SEQ ID NO:** |
|---|---|---|
| CDRH3 | FYSFAY | 33 |
| CDRL3 | SQSIHVPYT | 22 |

**Table 5C. CDR sequences of 52H5D3B8-3#**

| **52H5D3B8-3#** | **Sequence** | **SEQ ID NO:** |
|---|---|---|
| CDRH3 | FYSYAY | 20 |
| CDRL3 | FQSIHVPYT | 18 |

**Table 5D. CDR sequences of 52H5D3B8-4#**

| **52H5D3B8-4#** | **Sequence** | **SEQ ID NO:** |
|---|---|---|
| CDRH3 | FYSYAY | 20 |
| CDRL3 | SQSTHVPYT | 28 |

**Table 5E. CDR sequences of 52H5D3B8-5#**

| **52H5D3B8-5#** | **Sequence** | **SEQ ID NO:** |
|---|---|---|
| CDRH3 | YYSFAY | 24 |
| CDRL3 | SQSIHVPYT | 22 |

**Table SF. CDR sequences of 52H5D3B8-6#**

| **52H5D3B8-6#** | **Sequence** | **SEQ ID NO:** |
|---|---|---|
| CDRH3 | YYSYAY | 15 |
| CDRL3 | FQSIHVPYT | 18 |

**Table 5G. CDR sequences of 52H5D3B8-7#**

| **52H5D3B8-7#** | **Sequence** | **SEQ ID NO:** |
|---|---|---|
| CDRH3 | YYSYAY | 15 |
| CDRL3 | SQSTHVPYT | 28 |

**Table 5H. CDR sequences of 52H5D3B8-8#**

| **52H5D3B8-8#** | **Sequence** | **SEQ ID NO:** |
|---|---|---|
| CDRH3 | FYSFAY | 33 |
| CDRL3 | FQSIHVPYT | 18 |

**Table 51. CDR sequences of 52H5D3B8-9#**

| **52H5D3B8-9#** | **Sequence** | **SEQ ID NO:** |
|---|---|---|
| CDRH3 | FYSFAY | 33 |
| CDRL3 | SQSTHVPYT | 28 |

**Table 5J. CDR sequences of 52H5D3B8-10#**

| **52H5D3B8-10#** | **Sequence** | **SEQ ID NO:** |
|---|---|---|
| CDRH3 | FYSYAY | 20 |
| CDRL3 | FQSTHVPYT | 34 |

**Table 5K. CDR sequences of 52H5D3B8-11#**

| **52H5D3B8-11#** | **Sequence** | **SEQ ID NO:** |
|---|---|---|
| CDRH3 | FYSFAY | 33 |
| CDRL3 | FQSTHVPYT | 34 |

**Table 5L. CDR sequences of 52H5D3B8-12#**

| **52H5D3B8-12#** | **Sequence** | **SEQ ID NO:** |
|---|---|---|
| CDRH3 | YYSYAY | 15 |
| CDRL3 | FQSTHVPYT | 34 |

**Table SM. CDR sequences of 52H5D3B8-13#**

| **52H5D3B8-13#** | **Sequence** | **SEQ ID NO:** |
|---|---|---|
| CDRH3 | YYSFAY | 24 |
| CDRL3 | SQSTHVPYT | 28 |

**Table 5N. CDR sequences of 52H5D3B8-14#**

| **52H5D3B8-14#** | **Sequence** | **SEQ ID NO:** |
|---|---|---|
| CDRH3 | YYSFAY | 24 |
| CDRL3 | FQSIHVPYT | 18 |

**Table 5O. CDR sequences of 52H5D3B8-15#**

| **52H5D3B8-15#** | **Sequence** | **SEQ ID NO:** |
|---|---|---|
| CDRH3 | YYSFAY | 24 |
| CDRL3 | FQSTHVPYT | 34 |

### Cell binding of the optimized antibodies to GPC3-CHOK1 cells

Cell-based binding: FACS was used to evaluate the binding activity of 52H5D3B8, 52H5D3B8-1#, 52H5D3B8-2#, 52H5D3B8-3#, 52H5D3B8-4#, 52H5D3B8-5#, 52H5D3B8-6#, 52H5D3B8-7#, 52H5D3B8-8#, 52H5D3B8-9#, 52H5D3B8-10#, 52H5D3B8-11#, 52H5D3B8-12#, 52H5D3B8-13#, 52H5D3B8-14# and 52H5D3B8-15# chimeric mAbs on human GPC3 over-expressed CHOK1 cells.

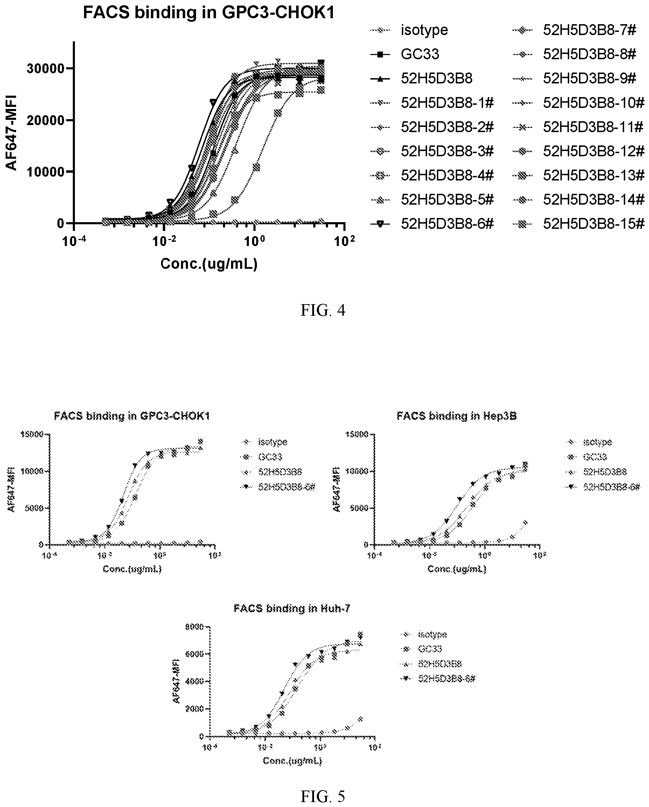

Briefly, GPC3-CHOK1 cells were firstly incubated with Fc blocking reagent at 4°C for 15 mins. Cells were washed by FACS buffer and divided to each well with 3-fold serially diluted 52H5D3B8, 52H5D3B8-1#, 52H5D3B8-2#, 52H5D3B8-3#, 52H5D3B8-4#, 52H5D3B8-5#, 52H5D3B8-6#, 52H5D3B8-7#, 52H5D3B8-8#, 52H5D3B8-9#, 52H5D3B8-10#, 52H5D3B8-11#, 52H5D3B8-12#, 52H5D3B8-13#, 52H5D3B8-14# and 52H5D3B8-15# chimeric mAbs starting at 30 µg/mL at 4°C for 60 mins. Cells were washed by FACS buffer and fixed with 2% PFA at RT for 15 mins. After washing by FACS buffer, Alexa Fluor^{®} 647 AffiniPure Goat Anti-Human IgG was added to each well and incubated at RT for 30 mins. Samples were washed twice with FACS buffer. The mean florescence intensity (MFI) of Alexa Fluor^{®} 647 was evaluated by MACS Quant Analyzer 16. As shown in **FIG. 4**, 52H5D3B8-6# chimeric mAbs bound to GPC3-CHOK1 cells with higher affinity than others. The EC50 of each chimeric mAbs on GPC3-CHOK1 cells is listed in **Table 6** below.

**Table 6. The binding activity to GPC3-CHOK1 cells and affinity ranking by Biacore**

| **EC50 mAbs** | **GPC3-CHOK1 cells** | **ka (1/Ms)** | **kd (1/s)** | **KD (M)** |
|---|---|---|---|---|
| **52H5D3B8** | 0.0705 µg/mL | | | |
| **52H5D3B8-1#** | 0.1041 µg/mL | 3.90E+05 | 2.88E-04 | **7.40E-10** |
| **52H5D3B8-2#** | 0.1486 µg/mL | 2.28E+05 | 1.21E-03 | **5.31E-09** |
| **52H5D3B8-3#** | 0.0869 µg/mL | 5.25E+05 | 5.56E-04 | **1.06E-09** |
| **52H5D3B8-4#** | 0.0986 µg/mL | 3.35E+05 | 5.78E-04 | **1.72E-09** |
| **52H5D3B8-5#** | 0.4051 µg/mL | 1.00E+05 | 4.12E-04 | **4.12E-09** |
| **52H5D3B8-6#** | 0.0601 µg/mL | 7.85E+05 | 2.89E-04 | **3.68E-10** |
| **52H5D3B8-7#** | 0.2324 µg/mL | 1.55E+05 | 6.17E-04 | **3.97E-09** |
| **52H5D3B8-8#** | 0.0919 µg/mL | 4.94E+05 | 1.00E-03 | **2.03E-09** |
| **52H5D3B8-9#** | 0.2473 µg/mL | 1.67E+05 | 2.16E-03 | **1.29E-08** |
| **52H5D3B8-10#** | 0.1758 µg/mL | 2.91E+05 | 1.18E-03 | **4.05E-09** |
| **52H5D3B8-11#** | 0.1042 µg/mL | 3.26E+05 | 3.54E-03 | **1.09E-08** |
| **52H5D3B8-12#** | 0.2348 µg/mL | 2.40E+05 | 1.33E-03 | **5.56E-09** |
| **52H5D3B8-13#** | 1.553 µg/mL | 4.96E+04 | 1.43E-03 | **2.89E-08** |
| **52H5D3B8-14#** | 0.0895 µg/mL | 4.81E+05 | 7.37E-04 | **1.53E-09** |
| **52H5D3B8-15#** | 0.1501 µg/mL | 2.67E+05 | 7.55E-03 | **2.82E-08** |

### Affinity ranking of the optimized antibodies by Biacore

The binding of the optimized antibodies of 52H5D3B8 mAb to human GPC3-his was tested with Biacore using a capture method. A single dose or two doses of human GPC3-his tag protein was injected over captured antibody. All the experiments were carried out on a Biacore 8K. Data analysis was carried out using Biacore 8K evaluation software.

As shown in the results of **Table 6** above, the optimized antibodies exhibited diverse binding affinity to the recombinant GPC3 protein ranging from 10⁻⁸ to 10⁻¹⁰. Besides, 52H5D3B8-6# mAbs showed the highest affinity to GPC3 protein.

### Cell binding of the optimized antibodies to GPC3-CHOK1 cells

Cell-based binding: FACS was used to evaluate the binding activity of 52H5D3B8, 52H5D3B8-6# chimeric mAbs and GC33 antibody on human GPC3 over-expressed CHOK1 cells, Hep3B cells and Huh-7 cells.

Briefly, GPC3-CHOK1 cells, Hep3B cells or Huh-7 cells were firstly incubated with Fc blocking reagent at 4°C for 15 mins. Cells were washed by FACS buffer and divided to each well with 3-fold serially diluted 52H5D3B8, 52H5D3B8-6# chimeric mAbs or GC33 antibody starting at 30 µg/mL at 4°C for 60 mins. Cells were washed by FACS buffer and fixed with 2% PFA at RT for 15 mins. After washing by FACS buffer, Alexa Fluor^{®} 647 AffiniPure Goat Anti-Human IgG was added to each well and incubated at RT for 30 mins. Samples were washed twice with FACS buffer. The mean florescence intensity (MFI) of Alexa Fluor^{®} 647 was evaluated by MACS Quant Analyzer 16. As shown in **FIG. 5**, 52H5D3B8-6# chimeric mAb bound to GPC3-CHOK1 cells, Hep3B cells and Huh-7 cells with higher affinity than both the 52H5D3B8 chimeric mAb and the GC33 antibody. The EC50 of each mAb on GPC3-CHOK1 cells, Hep3B cells and Huh-7 cells is listed in **Table 7** below.

**Table 7. The binding activity to GPC3-CHOK1 cells, Hep3B cells and Huh-7 cells**

| **EC50 mAbs** | **GPC3-CHOK1 cells** | **Hep3B cells** | **Huh-7 cells** |
|---|---|---|---|
| **52H5D3B8** | 0.0700 µg/mL | 0.2171 µg/mL | 0.0795 µg/mL |
| **52H5D3B8-6#** | 0.0474 µg/mL | 0.0982 µg/mL | 0.0501 µg/mL |
| **GC33** | 0.1308 µg/mL | 0.3754 µg/mL | 0.1469 µg/m L |

### Example 5. Humanization of the Chimeric Antibody 52H5D3B8-6#

The 52H5D3B8-6# variable regions were employed to create a humanized mAb. In the first step of this process, the amino acid sequences of the VH and VL of 52H5D3B8-6# were compared against the available database of human Ig gene sequences to find the overall best-matching human germline Ig gene sequences.

For the light chain of 52H5D3B8-6#, IGKV2-29*02 is the best fit germline, and for the heavy chain of 52H5D3B8-6#, IGHV1-46*01 was chosen as the humanization backbone. Humanized 52H5D3B8-6# CDR grafting antibody was then designed where the CDRL1, L2, and L3 were grafted onto framework sequences of the IGKV2-29*02, and the CDRH1, H2, and H3 were grafted onto framework sequences of the IGHV1-46*01. A 3D model was then generated to determine the amino acids in the original mouse FR region sequences that are essential for antibody binding and conformation. Based on the 52H5D3B8-6# CDR grafting antibody sequence, 3 additional humanized heavy chains and 3 additional light chains were created, In the case of the heavy chain, L, K, I, A, F and T in the framework were involved in back-mutations. In the case of the light chain, K and F in the framework were involved in back-mutations.

The amino acid sequences of the humanized antibodies are listed in **Table 8** below.

**Table 8A. Humanized antibody sequences (underlining indicates CDR; bold/italic indicates back mutations)**

| **52H5D3B8-6#** | **Sequence** | **SEQ ID NO:** |
|---|---|---|
| VH | QVQLQQSGAELVRPGASVKLSCKALGYTFTDYEMHWVKQTPVHGLEWIGA IHPGSGGTAYNQKFKGKATLTADKFSSTGYMELSSLTSEDSAVYYCTRYY SYAYWGQGTLVTVSA | 35 |
| VH1 | QVQLVQSGAEVKKPGASVKVSCKASGYTFTDYEMHWVRQAPGQGLEWMGA IHPGSGGTAYNQKFKGRVTMTRDTSTSTVYMELSSLRSEDTAVYYCARYY SYAYWGQGTLVTVSS | 36 |
| VH2 | QVQLVQSGAEVKKPGASVKVSCKASGYTFTDYEMHWVRQAPGQGLEWIGA IHPGSGGTAYNQKFKGRVTMTADTSTSTVYMELSSLRSEDTAVYYCTRYY SYAYWGQGTLVTVSS | 37 |
| VH3 | QVQLVQSGAEVKKPGASVKVSCKALGYTFTDYEMHWVKQAPGQGLEWIGA IHPGSGGTAYNQKFKGRVTLTADTSTSTVYMELSSLRSEDTAVYYCTRYY SYAYWGQGTLVTVSS | 38 |
| VH4 | QVQLVQSGAEVKKPGASVKVSCKALGYTFTDYEMHWVKQAPGQGLEWIGA IHPGSGGTAYNQKFKGRATMTADKFTSTVYMELSSLRSEDTAVYYCTRYY SYAYWGQGTLVTVSS | 39 |
| VL | DVVMTQTPLSLPVSLGDQASISCRSSQSLVHSNGNTYLQWYLQKPGQSPK LLIYKVSNRFSGVPDRFSGSGSGTDFILKISRVEAEDLGVYFCFQSIHVP YTFGGGTKLEIK | 40 |
| VL1 | DIVMTQTPLSLSVTPGQPASISCRSSQSLVHSNGNTYLQWYLQKPGQSPQ LLIYKVSNRFSGVPDRFSGSGSGTDFTLKISRVEAEDVGVYYCFQSIHVP YTFGGGTKLEIK | 41 |
| VL2 | DIVMTQTPLSLSVTPGQPASISCRSSQSLVHSNGNTYLQWYLQKPGQSPQ LLIYKVSNRFSGVPDRFSGSGSGTDFTLKISRVEAEDVGVYFCFQSIHVP YTFGGGTKLEIK | 42 |
| VL3 | DIVMTQTPLSLSVTPGQPASISCRSSQSLVHSNGNTYLQWYLQKPGQSPK LLIYKVSNRFSGVPDRFSGSGSGTDFTLKISRVEAEDVGVYYCFQSIHVP YTFGGGTKLEIK | 43 |
| VL4 | DIVMTQTPLSLSVTPGQPASISCRSSQSLVHSNGNTYLQWYLQKPGQSPK LLIYKVSNRFSGVPDRFSGSGSGTDFTLKISRVEAEDVGVYFCFQSIHVP YTFGGGTKLEIK | 44 |

**Table 8B. CDR sequences of 52H5D3B8-6#**

| **52H5D3B8-6#** | **Sequence** | **SEQ ID NO:** |
|---|---|---|
| CDRH1 | DYEMH | 13 |
| CDRH2 | AIHPGSGGTAYNQKFKG | 19 |
| CDRH3 | YYSYAY | 15 |
| CDRL1 | RSSQSLVHSNGNTYLQ | 21 |
| CDRL2 | KVSNRFS | 17 |
| CDRL3 | FQSIHVPYT | 18 |

Four humanized heavy chains and 4 humanized light chains were then synthesized and cloned into pcDNA3.4 vector respectively. After plasmids extraction, 4 heavy chains and 4 light chains were paired and expressed in HEK293 cells. The pairing of the human VH and the human VL created the 16 humanized antibodies (see Table 9).

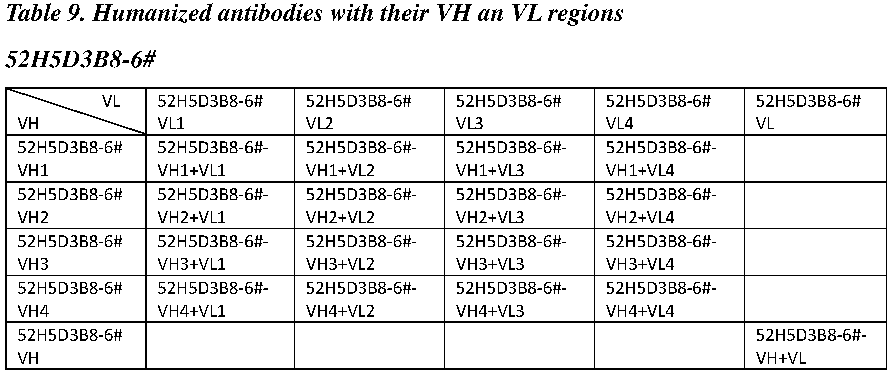

### Example 6: Antigen Binding Properties of the Humanized Antibodies

### Affinity ranking of the Humanized Antibodies by Biacore

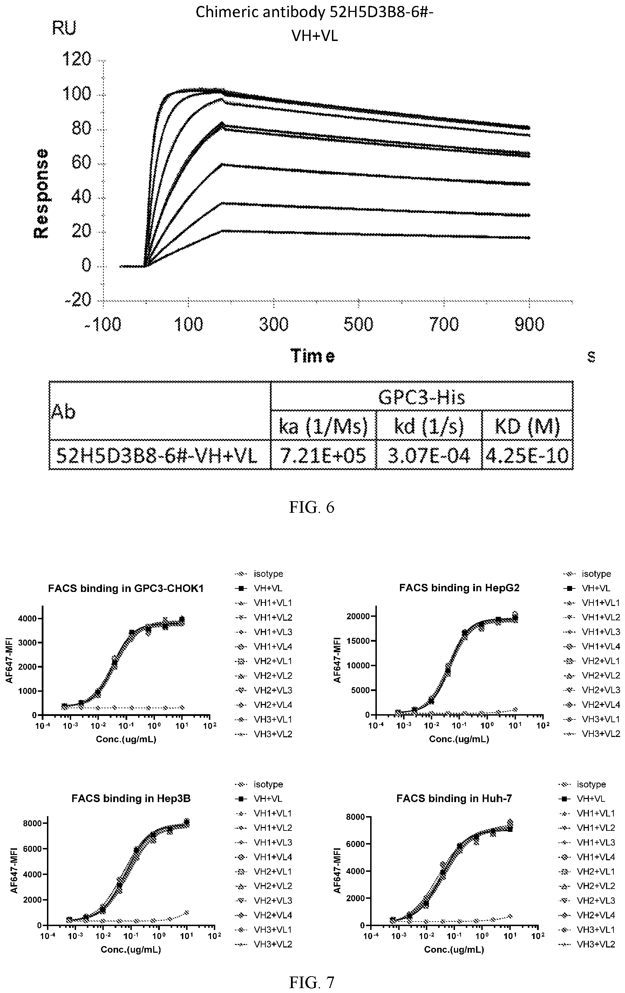

To evaluate antigen binding activity, the chimeric antibody's affinity was measured by Biacore 8K. A serial dilution of human GPC3-his tag protein was injected over captured chimeric antibody 52H5D3B8-6#-VH+VL for 3 mins at a flow rate of 30 µl/min. The antigen was allowed to dissociate for 720s. Among the multi detection doses, two concentrations with most close affinity to the chimeric antibody's affinity were chosen for the 16 humanized antibodies' affinity detection. As shown in **FIG. 6** and **Table 10,** all 16 humanized antibodies showed similar affinity, which are comparable to chimeric antibody.

**Table 10. Affinity ranking of humanized antibodies**

| **Abs** | **GPC3-His** | | | **Number of backmutation sites** |
|---|---|---|---|---|
| | **ka (1/Ms)** | **kd (1/s)** | **KD (M)** | |
| **52H5D3B8-6#-VH+VL** | 7.21E+05 | 3.07E-04 | **4.25E-10** | WT |
| **52H5D3B8-6#-VH1+VL1** | 5.67E+05 | 1.12E-03 | **1.97E-09** | 0+0 |
| **52H5D3B8-6#-VH1+VL2** | 5.64E+05 | 1.13E-03 | **2.00E-09** | 0+1 |
| **52H5D3B8-6#-VH1+VL3** | 5.61E+05 | 9.75E-04 | **1.74E-09** | 0+1 |
| **52H5D3B8-6#-VH1+VL4** | 5.46E+05 | 9.47E-04 | **1.74E-09** | 0+2 |
| **52H5D3B8-6#-VH2+VL1** | 6.46E+05 | 7.42E-04 | **1.15E-09** | 3+0 |
| **52H5D3B8-6#-VH2+VL2** | 6.51E+05 | 7.49E-04 | **1.15E-09** | 3+1 |
| **52H5D3B8-6#-VH2+VL3** | 6.68E+05 | 6.42E-04 | **9.62E-10** | 3+1 |
| **52H5D3B8-6#-VH2+VL4** | 6.48E+05 | 6.45E-04 | **9.94E-10** | 3+2 |
| **52H5D3B8-6#-VH3+VL1** | 6.89E+05 | 5.78E-04 | **8.38E-10** | 6+0 |
| **52H5D3B8-6#-VH3+VL2** | 6.64E+05 | 5.87E-04 | **8.83E-10** | 6+1 |
| **52H5D3B8-6#-VH3+VL3** | 7.14E+05 | 5.14E-04 | **7.21E-10** | 6+1 |
| **52H5D3B8-6#-VH3+VL4** | 7.11E+05 | 5.16E-04 | **7.25 E-10** | 6+2 |
| **52H5D3B8-6#-VH4+VL1** | 6.43E+05 | 6.46E-04 | **1.01E-09** | 8+0 |
| **52H5D3B8-6#-VH4+VL2** | 6.42E+05 | 6.74E-04 | **1.05E-09** | 8+1 |
| **52H5D3B8-6#-VH4+VL3** | 7.09E+05 | 6.01E-04 | **8.47E-10** | 8+1 |
| **52H5D3B8-6#-VH4+VL4** | 6.92E+05 | 5.93E-04 | **8.57E-10** | 8+2 |

### Binding to human GPC3 over-expressed CHOK1 cells, HEPG2 cells, Hep3B cells and Huh-7 cells

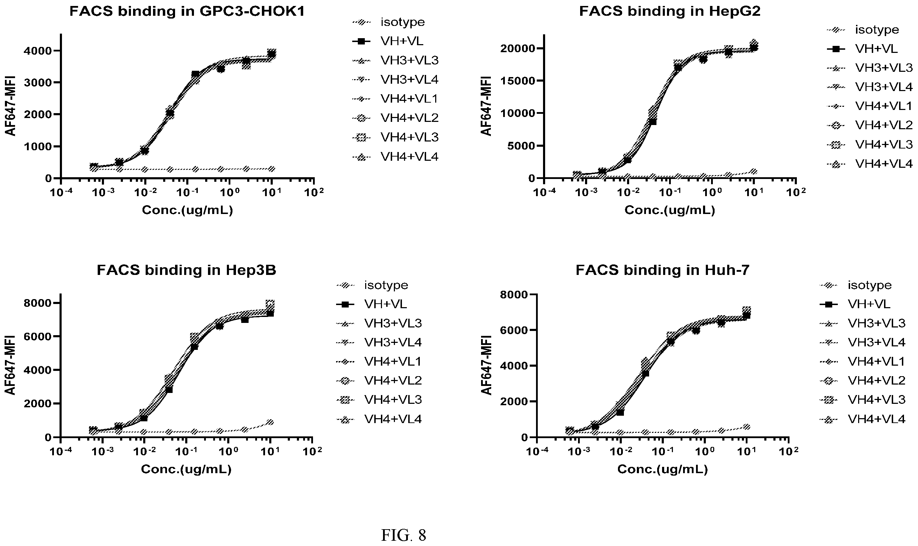

To evaluate the antigen binding property, the humanized antibodies were analyzed for their binding to human GPC3 over-expressed CHOK1 cells, HEPG2 cells, Hep3B cells and Huh-7 cells by FACS. Briefly, GPC3-CHOK1 cells, HEPG2 cells, Hep3B cells and Huh-7 cells were firstly incubated with Fc blocking reagent at 4°C for 15 mins. Cells were washed by FACS buffer and divided to each well with 4-fold serially diluted humanized antibodies starting at 10 µg/mL at 4°C for 60 mins. Cells were washed by FACS buffer and fixed with 2% PFA at RT for 15 mins. After washed by FACS buffer, the Alexa Fluor^{®} 647 AffiniPure Goat Anti-Human IgG was added to each well and incubated at RT for 30 mins. The MFI of Alexa Fluor^{®} 647 was evaluated by MACS Quant Analyzer 16. The 16 humanized antibodies were separated into two plates. As shown in **FIG. 7-8****.** The EC50 of each humanized antibody on GPC3-CHOK1 cells, HEPG2 cells, Hep3B cells and Huh-7 cells are listed in **Tables 11-12** below. By comparison of the EC50 of humanized antibodies with 52H5D3B8-6#-VH+VL in the same plate, all candidates of humanized antibodies showed comparable binding efficiency to the chimeric antibody.

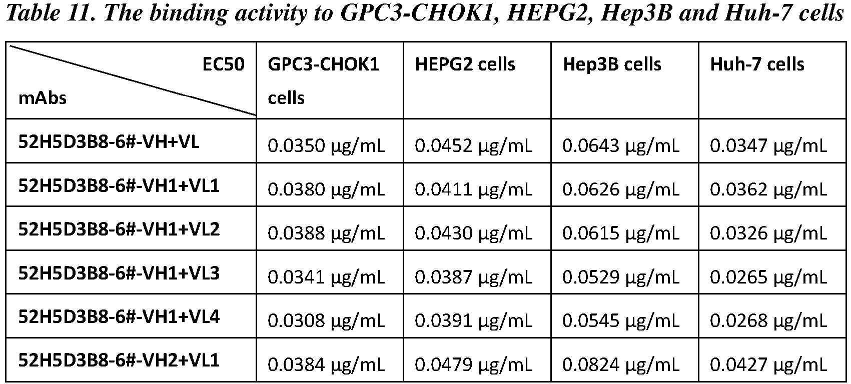

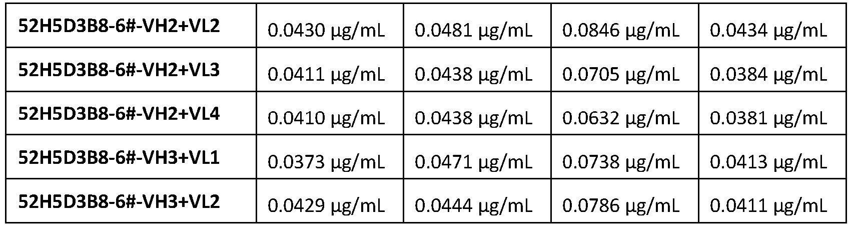

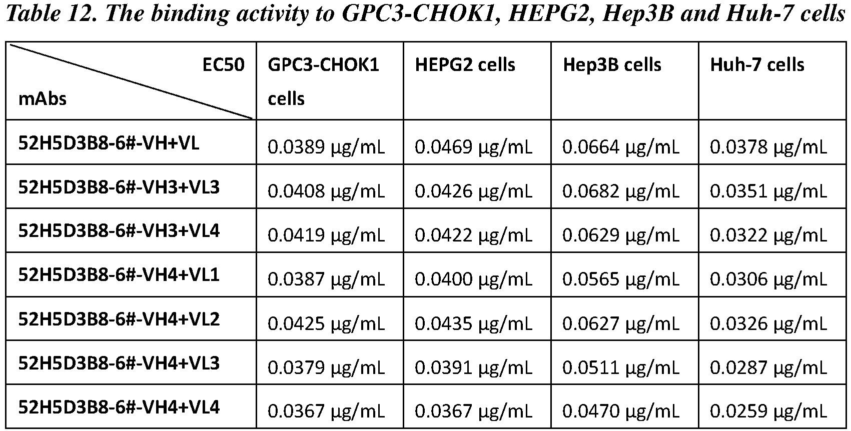

### Full kinetic affinity of humanized antibodies by Biacore

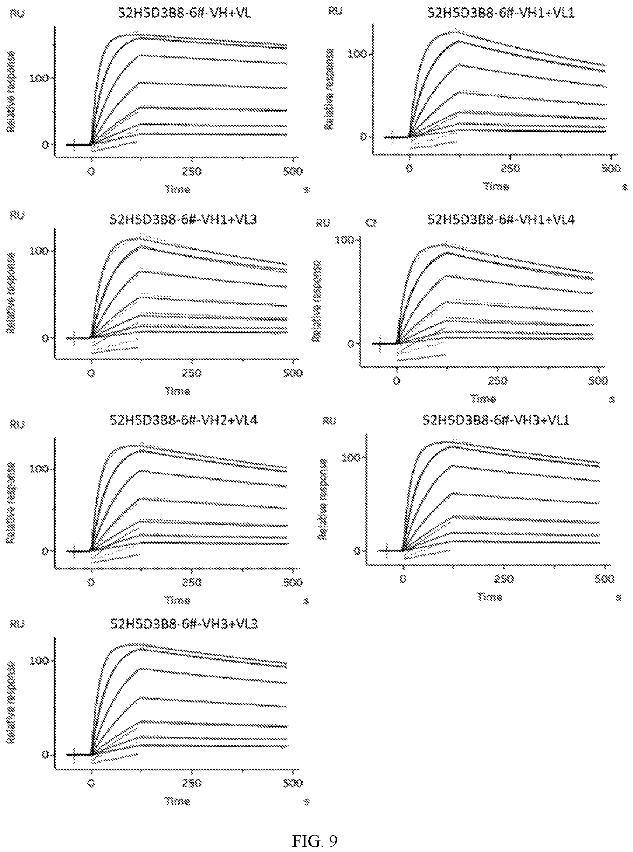

The binding of the humanized antibodies to recombinant GPC3 protein (human GPC3-his tag) was tested by Biacore using a capture method. 52H5D3B8-6#-VH+VL, 52H5D3B8-6#- VHI + VL 1, 52H5D3B8-6#- VHI + VL3, 52H5D3B8-6#- VHI + VL4, 52H5D3B8-6#-VH2+VL4, 52H5D3B8-6#-VH3+VL1, 52H5D3B8-6#-VH3+VL3 mAbs were captured using CM5 sensor chip. A serial dilution of human GPC3-his tag protein was injected over captured antibody for 2 mins at a flow rate of 30 µl/min. The antigen was allowed to dissociate for 360s. All the experiments were carried out on a Biacore T200. Data analysis was carried out using Biacore 8K evaluation software and the results are shown in **FIG. 9** and **Table 13** below.

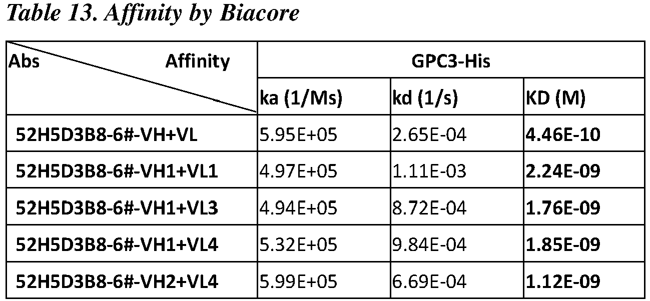

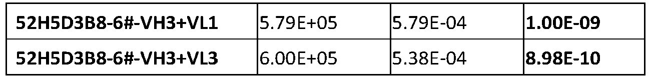

### Binding to human GPC3 over-expressed CHOK1 cells and HEPG2 cells

FACS was used to evaluate the binding activity of 52H5D3B8-6#-VH1+VL1 and 52H5D3B8-6#-VH2+VL3 with comparison to GC33 (Chugai Pharmaceutical) and Y035 (Carsgen Therapeutics, U.S. Patent Application US20190046659) on human GPC3 over-expressed CHOK1 cells and HEPG2 cells. The EC50 of each antibody on GPC3-CHOK1 cells and HEPG2 cells are listed in **Table 14** below.

As shown in **FIG. 10****,** both 52H5D3B8-6#-VH1+VL1 and 52H5D3B8-6#-VH2+VL3 bound to GPC3-CHOK1 cells and HEPG2 cells with comparable affinity, which are higher than benchmark mAbs.

**Table 14. The binding activity to GPC3-CHOKI and HEPG2 cells**

| **EC50 mAbs** | **GPC3-CHOK1 cells** | **HEPG2 cells** |
|---|---|---|
| **52H5D3B8-6#-VH1+VL1** | 0.3315 nM | 0.3416 nM |
| **52H5D3B8-6#-VH2+VL3** | 0.3436 nM | 0.3536 nM |
| **GC33** | 0.9556 nM | 0.7449 nM |
| **Y035** | 1.460 nM | 1.250 nM |

### Example 7: Affinity Maturation of Humanized Antibodies 52H5D3B8-6#

In order to enhance the affinity of humanized antibodies of 52H5D3B8-6#, affinity maturation was performed. Briefly, 4 phage libraries containing single-point or two-point saturation mutation in CDR regions were constructed. Three candidates with unique mutations in CDRs were obtained by 2 rounds of screening with solid or liquid panning. CDRs of these candidates were engrafted into 52H5D3B8-6#-VH2+VL3 frameworks to generate antibodies for further binding and functional validation.

The amino acid sequences of the variable regions of frameworks engrafted affinity maturated candidates are listed in **Table 15** below.

**Table 15. Sequences of the variable regions of 52H5D3B8-18#-VH2+VL3, 52H5D3B8-19#-VH2+VL3, 52H5D3B8-20#-VH2+VL3**

| **Name** | **Sequence** | **SEQ ID NO:** |
|---|---|---|
| 52H5D3B8-18#-VH2+VL3 VH | QVQLVQSGAEVKKPGASVKVSCKASGYTFTRYEMHWVRQAPGQGLEWIGA IHPGSGGTAYNSKFKGRVTMTADTSTSTVYMELSSLRSEDTAVYYCTRYY SYAYWGQGTLVTVSS | 53 |
| 52H5D3B8-18#-VH2+VL3 VL | DIVMTQTPLSLSVTPGQPASISCRSSQSLVHSNGNTYLQWYLQKPGQSPK LLIYKVSNRYSGVPDRFSGSGSGTDFTLKISRVEAEDVGVYYCVESIHVP YTFGGGTKLEIK | 54 |
| 52H5D3B8-19#-VH2+VL3 VH | QVQLVQSGAEVKKPGASVKVSCKASGYTFTGYEMHWVRQAPGQGLEWIGA IHPGSGGTAYNQKFKGRVTMTADTSTSTVYMELSSLRSEDTAVYYCTRYY SYAYWGQGTLVTVSS | 55 |
| 52H5D3B8-19#-VH2+VL3 VL | DIVMTQTPLSLSVTPGQPASISCRSSQSLVHSNGNTYLQWYLQKPGQSPK LLIYKVSNRFAGVPDRFSGSGSGTDFTLKISRVEAEDVGVYYCVESIHVP YTFGGGTKLEIK | 56 |
| 52H5D3B8-20#-VH2+VL3 VH | QVQLVQSGAEVKKPGASVKVSCKASGYRFTDYEMHWVRQAPGQGLEWIGA IHPGSGGTAYNQLFKGRVTMTADTSTSTVYMELSSLRSEDTAVYYCTRYY SYAYWGQGTLVTVSS | 57 |
| 52H5D3B8-20#-VH2+VL3 VL | DIVMTQTPLSLSVTPGQPASISCRSSQSLVHSNGNTYLQWYLQKPGQSPK LLIYKVSNRFPGVPDRFSGSGSGTDFTLKISRVEAEDVGVYYCVESIHVP YTFGGGTKLEIK | 58 |

**Table 15A. CDR sequences of 52H5D3B8-18#-VH2+VL3**

| **52H5D3B8-18#-VH2+VL3** | **Sequence** | **SEQ ID NO:** |
|---|---|---|
| CDRH1 | RYEMH | 59 |
| CDRH2 | AIHPGSGGTAYNSKFKG | 60 |
| CDRH3 | YYSYAY | 15 |
| CDRL1 | RSSQSLVHSNGNTYLQ | 21 |
| CDRL2 | KVSNRYS | 61 |
| CDRL3 | VESIHVPYT | 62 |

**Table 15B. CDR sequences of 52H5D3B8-19#-VH2+VL3**

| **52H5D3B8-19#-VH2+VL3** | **Sequence** | **SEQ ID NO:** |
|---|---|---|
| CDRH1 | GYEMH | 63 |
| CDRH2 | AIHPGSGGTAYNQKFKG | 19 |
| CDRH3 | YYSYAY | 15 |
| CDRL1 | RSSQSLVHSNGNTYLQ | 21 |
| CDRL2 | KVSNRFA | 64 |
| CDRL3 | VESIHVPYT | 62 |

**Table 15C. CDR sequences of 52H5D3B8-20#-VH2+VL3**

| **52H5D3B8-20#-VH2+VL3** | **Sequence** | **SEQ ID NO:** |
|---|---|---|
| CDRH1 | DYEMH | 13 |
| CDRH2 | AIHPGSGGTAYNQLFKG | 65 |
| CDRH3 | YYSYAY | 15 |
| CDRL1 | RSSQSLVHSNGNTYLQ | 21 |
| CDRL2 | KVSNRFP | 66 |
| CDRL3 | VESIHVPYT | 62 |

### Binding to human GPC3 over-expressed CHOK1 cells

To evaluate the antigen binding property, the affinity maturated antibodies were analyzed for their binding to human GPC3 over-expressed CHOK1 cells by FACS as previously described. The EC50 of each antibody on GPC3-CHOK1 cells are listed in **Table 16** below.

As shown in **FIG. 11****,** all affinity maturated antibodies showed enhanced binding efficiency on GPC3-CHOK1 cells than the parental 52H5D3B8-6#-VH2+VL3 and benchmark antibody GC33.

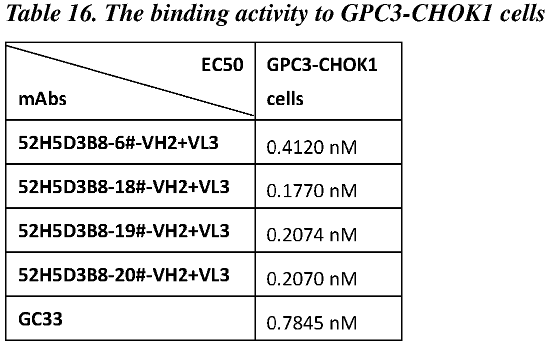

### Example 8: Humanized GPC3 Antibodies Mediated ADCC Signaling

To evaluate antigen binding mediated antibody-dependent cell cytotoxicity (ADCC) signaling of 52H5D3B8-6#- VHI + VL 1, 52H5D3B8-6#-VH2+VL3 and three affinity maturated antibodies with comparison to GC33, a ADCC signaling reporter assay was set up. Briefly, full length human CD16a with high affinity allotype (176V) binding to Fc region was introduced into a Jurkat-NFAT luciferase reporter cell line (referred to as Jukrat-hCD16a-NFAT). The expression of a luciferase gene is under the control of the NFAT response element in this cell line. Jukrat-hCD16a-NFAT cell line allowed us to evaluate GPC3 binding mediated ADCC signaling by examining downstream NFAT signaling. This reporter cell line was cocultured with several GPC3 positive tumor cell lines including HEPG2 or Huh-7 hepatocellular carcinoma cells with high or moderate GPC3 expression level, respectively. The Luciferase-based chemiluminescence could be detected by ONE-Glo^{™} Luciferase assay system (Promega, Cat.No E6130) and Envision multilabel plate readers (PerkinElemer).

As shown in **FIG. 12** and **Table 17,** both 52H5D3B8-6#-VH1+VL1 and 52H5D3B8-6#-VH2+VL3 showed efficient GPC3 binding mediated ADCC signaling to HEPG2 cells and Huh-7 cells, which are higher than GC33. Besides, both 52H5D3B8-19#-VH2+VL3 and 52H5D3B8-20#-VH2+VL3 showed better EC50 of ADCC signaling to HEPG2 cells than parental 52H5D3B8-6#-VH2+VL3, while 52H5D3B8-18#-VH2+VL3 showed better maximum ADCC signaling potency than other antibodies. Of note, in Huh-7 cells which showed moderate GPC3 expression level, all humanized antibodies exhibited enhanced maximum ADCC signaling potency over GC33.

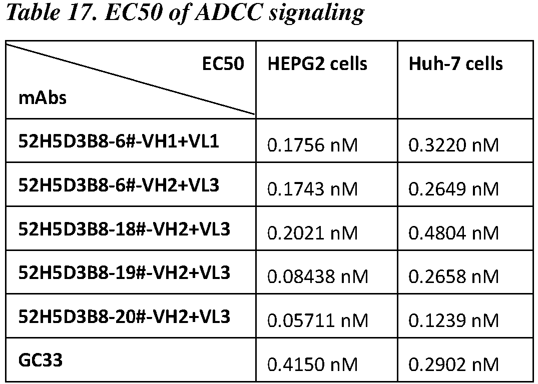

### Example 9: Humanized GPC3 Antibodies Mediated Tumor Killing by NK Cells

In this example, the functional activities of GPC3 humanized antibodies were tested.

### 9.1 NK cytotoxicity to target cells mediated by GPC3 humanized antibodies

To evaluate whether GPC3 mediated ADCC signaling activation could translate into a cytotoxic effect of NK cells on target cells, a human primary NK cell-mediated cytotoxicity assay was set-up. Briefly, fresh human primary NK cells (CD3-CD56+) were isolated from buffy coat of healthy donors by negative selection with magnetic beads (Miltenyi, Cat. No. 130-092-657). The purity of the isolated NK cells was monitored by FACS analysis and typically more than 90%. The isolated NK cells were then rested in the complete culture media for 24 hours, which were used as effector cells. HEPG2 or Huh-7 human hepatocellular carcinoma cell line were used as target cells. The NK cell mediated cytotoxicity assay was carried out by following the manufacturer's protocol of Cytotoxicity Detection Kit (Roche, Cat.No 11644793001). The effector cells at a density of 5.0×10⁴ cells per well were co-cultured with 1.0×10⁴ target cells (E/T Ratio = 5:1) in round bottom 96-well microplate. Antibodies were serially diluted at a 5-fold starting from 50 nM and added to corresponding wells. After 5 hours (for HEPG2 cells) or 24 hours (for Huh-7 cells) incubation at 37 °C, lactate dehydrogenase (LDH) representing cellular viability in each well were detected by the Cytotoxicity Detection Kit. Absorbance was measured at 492 nm. Formulas for calculating: Cytotoxicity (%) = (effector - target cell mix - effector cell control - low control) / (high control - low control) x 100. Four parameter nonlinear regression curve fit was performed with GraphPad Prism 9.

As shown in **FIG.** 13 and **Table 18,** both 52H5D3B8-6#-VH1+VL1 and 52H5D3B8-6#-VH2+VL3 showed efficient GPC3 antibody-mediated NK cytotoxicity to HEPG2 cells and Huh-7 cells, which are higher than GC33.

Besides, both 52H5D3B8-19#-VH2+VL3 and 52H5D3B8-20#-VH2+VL3 showed comparable GPC3 antibody-mediated NK cytotoxicity to HEPG2 cells with parental 52H5D3B8-6#-VH2+VL3. Interestingly, in Huh-7 cells which showed moderate GPC3 expression level, both 52H5D3B8-19#-VH2+VL3 and 52H5D3B8-20#-VH2+VL3 showed better EC50 than parental 52H5D3B8-6#-VH2+VL3.

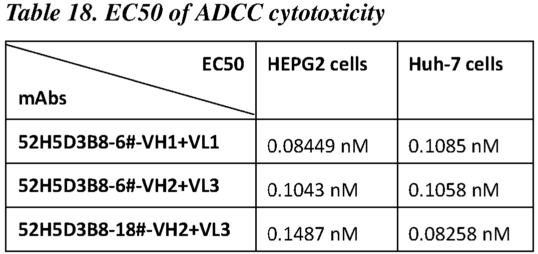

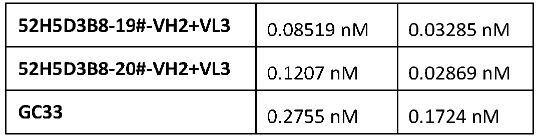

### 9.2 Degranulation of NK cells stimulated by GPC3 humanized antibodies

To investigate the ability of the GPC3 humanized antibodies to stimulate degranulation of NK cells, expression level of CD107a, a degranulation marker, was analyzed in NK cells. Briefly, human primary NK cells generated as described above as effector cells at a density of 2.5×10⁴ cells per well were co-incubated with 2.5x10⁴ target cells including HEPG2 (E/T Ratio = 1:1) in round bottom 96-well microplate. Antibodies were serially diluted at a 5-fold starting from 50 nM and added into corresponding wells. After 4 hours incubation at 37°C, the cells were harvested and washed with ice-cold staining buffer (0.5% BSAin PBS). The Anti-CD107a antibody (BioLegend, Cat. No 328608) was diluted in the staining buffer with 1 µL per well and incubated with the cells for 40 mins at 4°C in the dark. The samples were then washed and fixed with 2% PFA. Cells were analyzed on the flow cytometer MACSQuant^{®} Analyze 16 (Miltenyi Biotech). Data were analyzed with Flowjo 10.0 software. Four parameter nonlinear regression curve fit was performed with GraphPad Prism 9.

As shown in **FIG. 14****,** all GPC3 humanized antibodies stimulated more effective CD107a up-regulation than GC33 in the present of HEPG2 cells.

### 9.3 Cytokine production of NK stimulated by GPC3 humanized antibodies

To investigate the ability of the humanized antibodies to stimulate the cytokine production of NK cells in the presence of target cells, intercellular IFN-γ and TNF-α in NK cells were analyzed with FACS staining. Briefly, human primary NK cells were prepared as described above as effector cells at a density of 2.5x10⁴ cells per well were cocultured with 2.5x10⁴ target cells including HEPG2 (E/T Ratio = 1:1) in round bottom 96-well microplate. The protein trafficking inhibitor Brefeldin A (BFA) was added into the co-culture systems to prevent cytokine secretion to the supernatant during NK cell activation. Antibodies were serially diluted at a 5-fold starting from 50 nM and added into corresponding wells. After 4 hours incubation at 37°C, the samples were washed and intracellular staining following the procedure of Foxp3/Transcription Factor Staining Buffer Set (ThermoFisher, Cat.No 00-5523-00). The Anti-IFN-γ antibody (BioLegend, Cat. No 506510) and Anti-TNF-α antibody (BD Biosciences, Cat. No 562783) were diluted in the recommended staining buffer with 1 µL per well. Cells were analyzed on the flow cytometer MACSQuant^{®} Analyze 16 (Miltenyi Biotech). Data were analyzed with Flowjo 10.0 software. Four parameter nonlinear regression curve fit was performed with GraphPad Prism 9.

As shown in **FIG. 15****,** all GPC3 humanized antibodies induced more production of IFN-γ and the TNF-α in NK cells than GC33 in the present of HEPG2 cells.

### Example 10. Humanization of the Chimeric Antibody 52H5D3B8

The 52H5D3B8 variable region genes were employed to create a humanized mAb. In the first step of this process, the amino acid sequences of the VH and VL or VK of 52H5D3B8 were compared against the available database of human Ig gene sequences to find the overall best-matching human germline Ig gene sequences.

Briefly, IGKV2-29*02 and IGHV1-2*02 were used as backbone of heavy chain and light chain separately. A 3D model was then generated to determine the amino acids in the original mouse FR region sequences that are essential for antibody binding and conformation. Based on the 52H5D3B8 CDR grafting antibody sequence, 5 additional humanized heavy chains and 1 additional light chain were created, In the case of the heavy chain, I, K, A, L, T in the framework were involved in back-mutations. In the case of the light chain, F in the framework were involved in back-mutations.

The amino acid and nucleotide sequences of some of the humanized antibodies are listed in **Table 19** below.

**Table 19. Humanized antibody sequences (underlining indicates CDR; bold/italic indicates back mutations)**

| **52H5D3B8** | **Sequence** | **SEQ ID NO:** |
|---|---|---|
| VH | QVQLQQSGAELVRPGASVKLSCKALGYTFTDYEMHWVKQTPVHGLEWIGA IHPGSGGTAYNQKFKGKATLTADKFSSTGYMELSSLTSEDSAVYYCTRFY SYAYWGQGTLVTVSA | 3 |
| VHO | EVQLVQSGAEVKKPGASVKVSCKASGYTFTDYEMHWVRQAPGQGLEWMGA IHPGSGGTAYNQKFKGRVTMTRDTSISTAYMELSRLRSDDTAVYYCARFY SYAYWGQGTLVTVSS | 45 |
| VH1 | EVQLVQSGAEVKKPGASVKVSCKASGYTFTDYEMHWVRQAPGQGLEWMGA IHPGSGGTAYNQKFKGRVTMTRDTSISTAYMELSRLRSDDTAVYYCTRFY SYAYWGQGTLVTVSS | 46 |
| VH2 | EVQLVQSGAEVKKPGASVKVSCKASGYTFTDYEMHWVRQAPGQGLEWMGA IHPGSGGTAYNQKFKGRVTMTADTSISTAYMELSRLRSDDTAVYYCTRFY SYAYWGQGTLVTVSS | 47 |
| VH3 | EVQLVQSGAEVKKPGASVKVSCKASGYTFTDYEMHWVRQAPGQGLEWMGA IHPGSGGTAYNQKFKGRVTMTADKSISTAYMELSRLRSDDTAVYYCTRFY SYAYWGQGTLVTVSS | 48 |
| VH4 | EVQLVQSGAEVKKPGASVKVSCKASGYTFTDYEMHWVRQAPGQGLEWIGA IHPGSGGTAYNQKFKGRVTLTADKSISTAYMELSRLRSDDTAVYYCTRFY SYAYWGQGTLVTVSS | 49 |
| VH5 | EVQLVQSGAEVKKPGASVKVSCKASGYTFTDYEMHWVRQAPGQGLEWIGA IHPGSGGTAYNQKFKGKATLTADKSISTAYMELSRLRSDDTAVYYCTRFY SYAYWGQGTLVTVSS | 50 |
| VL | DVVMTQTPLSLPVSLGDQASISCRSSQSLVHSNGNTYLQWYLQKPGQSPK LLIYKVSNRFSGVPDRFSGSGSGTDFILKISRVEAEDLGVYFCSQSIHVP YTFGGGTKLEIK | 4 |
| VLO | DVVMTQTPLSLSVTPGQPASISCRSSQSLVHSNGNTYLQWYLQKPGQSPQ LLIYKVSNRFSGVPDRFSGSGSGTDFTLKISRVEAEDVGVYYCSQSIHVP YTFGGGTKVEIK | 51 |
| VL1 | DVVMTQTPLSLSVTPGQPASISCRSSQSLVHSNGNTYLQWYLQKPGQSPQ LLIYKVSNRFSGVPDRFSGSGSGTDFTLKISRVEAEDVGVYFCSQSIHVP YTFGGGTKVEIK | 52 |

Six humanized heavy chains and two humanized light chains were then synthesized and cloned into pcDNA3.4 vector respectively. After plasmids extraction, 6 heavy chains and 2 light chains were paired and expressed in HEK293 cells. The pairing of the human VH and the human VL created the 12 humanized antibodies (see Table 20).

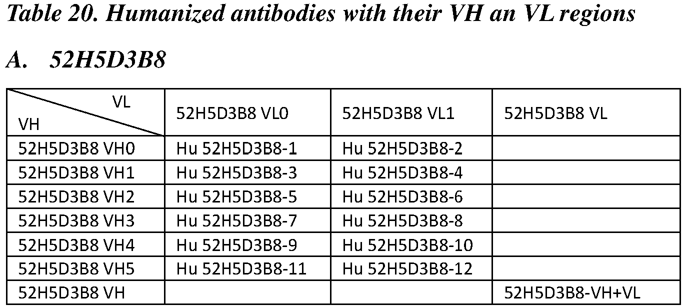

### Example 11: Antigen Binding Properties of the Humanized Antibodies

### Affinity ranking of the Humanized Antibodies by Biacore

To evaluate antigen binding activity, the chimeric antibody's affinity was measured by Biacore 8K. Two concentrations with most close affinity to the chimeric antibody's affinity were chosen for the 12 humanized antibodies' affinity detection. Human GPC3-his tag protein was injected over captured antibody for 3 mins at a flow rate of 30 µl/min. The antigen was allowed to dissociate for 720s. As shown in Table 21, all 12 humanized antibodies showed similar affinity to the chimeric antibody.

**Table 21. Affinity ranking of humanized antibodies**

| **Abs** | **GPC3-His** | | | **Number of backmutation sites** |
|---|---|---|---|---|
| | **ka (1/Ms)** | **kd (1/s)** | **KD (M)** | |
| **52H5D3B8-VH+VL** | 3.47E+05 | 4.90E-04 | **1.41E-09** | WT |
| **Hu 52H5D3B8-1** | 2.55E+05 | 1.61E-03 | **6.33E-09** | 0+0 |
| **Hu 52H5D3B8-2** | 2.85E+05 | 1.63E-03 | **5.71E-09** | 0+1 |
| **Hu 52H5D3B8-3** | 4.05E+05 | 1.76E-03 | **4.34E-09** | 1+0 |
| **Hu 52H5D3B8-4** | 3.57E+05 | 1.64E-03 | **4.60E-09** | 1+1 |
| **Hu 52H5D3B8-5** | 3.19E+05 | 9.16E-04 | **2.87E-09** | 2+0 |
| **Hu 52H5D3B8-6** | 3.27E+05 | 9.61E-04 | **2.94E-09** | 2+1 |
| **Hu 52H5D3B8-7** | 3.20E+05 | 1.03E-03 | **3.23E-09** | 3+0 |
| **Hu 52H5D3B8-8** | 3.32E+05 | 1.10E-03 | **3.31E-09** | 3+1 |
| **Hu 52H5D3B8-9** | 2.44E+05 | 1.02E-03 | **4.18E-09** | 5+0 |
| **Hu 52H5D3B8-10** | 2.67E+05 | 1.04E-03 | **3.89E-09** | 5+1 |
| **Hu 52H5D3B8-11** | 2.95E+05 | 9.05E-04 | **3.06E-09** | 7+0 |
| **Hu 52H5D3B8-12** | 3.10E+05 | 1.00E-03 | **3.22E-09** | 7+1 |

### Binding to human GPC3 over-expressed CHOKI cells and Huh-7 cells

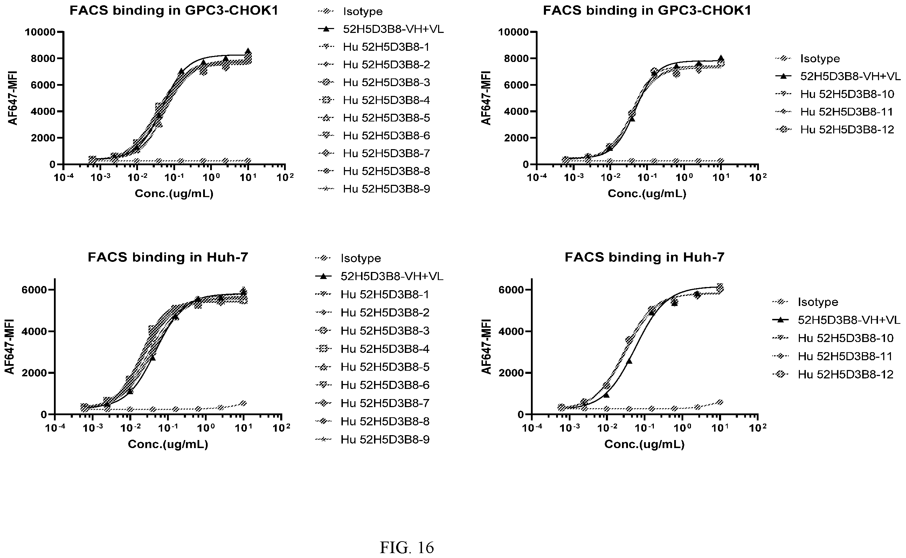

To evaluate the antigen binding property, the humanized antibodies were analyzed for their binding to human GPC3 over-expressed CHOK1 cells and Huh-7 cells by FACS. Briefly, GPC3-CHOK1 cells and Huh-7 cells were firstly incubated with Fc blocking reagent at 4°C for 15 mins. Cells were washed by FACS buffer and divided to each well with 4-fold serially diluted humanized antibodies starting at 10 µg/mL at 4°C for 60 mins. Cells were washed by FACS buffer and fixed with 2% PFA at RT for 15 mins. After washed by FACS buffer, the Alexa Fluor^{®} 647 AffiniPure Goat Anti-Human IgG was added to each well and incubated at RT for 30 mins. The MFI of Alexa Fluor^{®} 647 was evaluated by MACSQuant Analyzer 16. As different plates might affect the EC50 of 52H5D3B8-VH+VL, the 12 humanized antibodies were separated into two plates. The EC50 of each humanized antibody on GPC3-CHOK1 cells and Huh-7 cells are listed in **Tables 22-23** below. As shown in **FIG. 16****,** all candidates of humanized antibodies showed comparable binding efficiency to the chimeric antibody on GPC3-CHOK1 cells and better binding efficiency than the chimeric antibody on Huh-7 cells.

**Table 22. The binding activity to GPC3-CHOK1 and Huh-7 cells**

| **EC50 mAbs** | **GPC3-CHOK1 cells** | **Huh-7 cells** |
|---|---|---|
| **52H5D3B8-VH+VL** | 0.0477 µg/mL | 0.0469 µg/mL |
| **Hu 52H5D3B8-1** | 0.0404 µg/mL | 0.0243 µg/mL |
| **Hu 52H5D3B8-2** | 0.0338 µg/mL | 0.0195 µg/mL |
| **Hu 52H5D3B8-3** | 0.0387 µg/mL | 0.0213 µg/mL |
| **Hu 52H5D3B8-4** | 0.0324 µg/mL | 0.0196 µg/mL |
| **Hu 52H5D3B8-5** | 0.0544 µg/mL | 0.0402 µg/mL |
| **Hu 52H5D3B8-6** | 0.0509 µg/mL | 0.0295 µg/mL |
| **Hu 52H5D3B8-7** | 0.0447 µg/mL | 0.0242 µg/mL |
| **Hu 52H5D3B8-8** | 0.0432 µg/mL | 0.0233 µg/mL |
| **Hu 52H5D3B8-9** | 0.0572 µg/mL | 0.0348 µg/mL |

**Table 23. The binding activity to GPC3-CHOK1 and Huh-7 cells**

| **EC50 mAbs** | **GPC3-CHOK1 cells** | **Huh-7 cells** |
|---|---|---|
| **52H5D3B8-VH+VL** | 0.0479 µg/mL | 0.0563 µg/mL |
| **Hu 52H5D3B8-10** | 0.0389 µg/mL | 0.0323 µg/mL |
| **Hu 52H5D3B8-11** | 0.0400 µg/mL | 0.0288 µg/mL |
| **Hu 52H5D3B8-12** | 0.0384 µg/mL | 0.0294 µg/mL |

### Binding to human GPC3 over-expressed CHOKI cells, Huh-7 cells and HEPG2 cells

FACS was used to evaluate the binding activity of Hu 52H5D3B8-7 and Hu 52H5D3B8-8 with comparison to benchmark antibodies GC33 and Y035 on human GPC3 expressed on CHOK1 cells, Huh-7 cell and HEPG2 cells. In order to evaluate the properties of CDRs and frameworks of Hu 52H5D3B8-7 Ab, two chimeric antibodies were generated. The antibody termed "Hu 52H5D3B8-7/8 CDRs with Y035 FRs" contains the Hu 52H5D3B8-7 CDRs and the Y035 FRs. The antibody termed "Y035 CDRs with Hu 52H5D3B8-7 FRs" represents the chimeric antibody with the Y035 CDRs engrafted to Hu 52H5D3B8-7 FRs. The EC50 of each antibody on GPC3-CHOK1 cells, Huh-7 cells and HEPG2 cells are listed in **Table 24** below.

As shown in **FIG. 17****,** both Hu 52H5D3B8-7 and Hu 52H5D3B8-8 bound to GPC3-CHOK1 cells, Huh-7 cells and HEPG2 cells with comparable affinity, which are higher than benchmark antibodies GC33 and Y035. More importantly, both Hu 52H5D3B8-7/8 CDRs with Y035 FRs and Y035 CDRs with Hu 52H5D3B8-7 FRs showed higher affinity than Y035, which demonstrated that the CDRs and frameworks of Hu 52H5D3B8-7 contributed to a superior binding ability to Y035.

**Table 24. The binding activity to GPC3-CHOKI, Huh-7 cells and HEPG2 cells**

| **EC50 mAbs** | **GPC3-CHOK1 cells** | **Huh-7 cells** | **HEPG2 cells** |
|---|---|---|---|
| **Hu 52H5D3B8-7** | 0.4674 nM | 0.4637 nM | 0.7574 nM |
| **Hu 52H5D3B8-8** | 0.4341 nM | 0.4177 nM | 0.6934 nM |
| **GC33** | 0.9925 nM | 0.9528 nM | 1.282 nM |
| **Y035** | 1.417 nM | 1.178 nM | 1.591 nM |
| **Hu 52H5D3B8-7/8 CDRs with Y035 FRs** | 0.7981 nM | 0.7824 nM | 1.162 nM |
| **Y035 CDRs with Hu 52H5D3B8-7 FRs** | 0.9854 nM | 0.6674 nM | 1.047 nM |

### Example 12: Internalization Activity of Humanized GPC3 Atnibodies on GPC3-expressed Tumor Cell Line

Cell-based internalization was used to evaluate the internalization activity of Hu 52H5D3B8-7 and Hu 52H5D3B8-8 with comparison to Y035 on HEPG2 cells. In order to evaluate the properties of CDRs and frameworks of Hu 52H5D3B8-7 Ab, two antibodies (Hu 52H5D3B8-7/8 CDRs with Y035 FRs and Y035 CDRs with Hu 52H5D3B8-7 FRs) were also evaluated.

PHAb thiol Dyes are pH sensor dyes that have very low fluorescence at pH > 7, and a dramatic increase in fluorescence will occur when as it is internalized into the endosome or lysosome where the pH is around 6.3 or 4.7, respectively. Briefly, a pHAb thiol Dyes labeled α-hIgG secondary antibody (10 µg/mL) was incubated with antibodies (20 µg/mL) for 30min. After incubation, the mixture Abs were serially diluted with 2-fold. Then the diluted mixture was added into 96-well assay plates pre-seeded with 2×10⁴ HEPG2 cells in each well. After 48 hours incubation, fluorescence signal was captured on Multimode Plate Reader (Envision^{®} 2105).

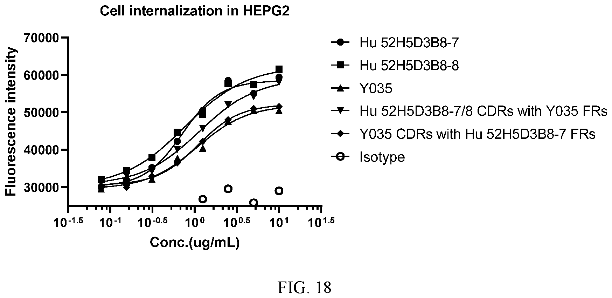

As shown in **FIG. 18****,** both Hu 52H5D3B8-7 and Hu 52H5D3B8-8 showed higher internalization efficacy than other mAbs. Besides, Hu 52H5D3B8-7/8 CDRs with Y035 FRs showed higher internalization efficacy than Y035 (higher Top and comparable EC50), which indicated that the CDRs of Hu-52H5D3B8-7 contribute to an enhanced target mediated internalization when compared with Y035. The EC50 and Top of each mAbs is listed in **Table 25** below.

**Table 25. The internalization activity to HEPG2 cells**

| **mAbs** | **EC50** | **Top** |
|---|---|---|
| **Hu 52H5D3B8-7** | 0.7195 µg/mL | 58555 |
| **Hu 52H5D3B8-8** | 0.7805 ug/mL | 62378 |
| **Y035** | 1.078 µg/mL | 51973 |
| **Hu 52H5D3B8-7/8 CDRs with Y035** FRs | 1.142 ug/mL | 59223 |
| **Y035 CDRs with Hu 52H5D3B8-7 FRs** | 1.107 ug/mL | 52157 |

The present disclosure is not to be limited in scope by the specific embodiments described which are intended as single illustrations of individual aspects of the disclosure, and any compositions or methods which are functionally equivalent are within the scope of this disclosure. It will be apparent to those skilled in the art that various modifications and variations can be made in the methods and compositions of the present disclosure without departing from the spirit or scope of the disclosure. Thus, it is intended that the present disclosure cover the modifications and variations of this disclosure provided they come within the scope of the appended claims and their equivalents.

All publications and patent applications mentioned in this specification are herein incorporated by reference to the same extent as if each individual publication or patent application was specifically and individually indicated to be incorporated by reference.

## Claims

1. An anti-glypican 3 (GPC3) antibody or fragment thereof which has specificity to the human glypican 3 protein and comprises a heavy chain variable region (VH) comprising a VH CDR1, a VH CDR2 and a VH CDR3, and a light chain variable region (VL) comprising a VL CDR1, a VL CDR2, and a VL CDR3, wherein the VH CDR1, VH CDR2, VH CDR3, VL CDR1, VL CDR2, and VL CDR3, respectively, comprise the amino acid sequences of SEQ ID NO: 13, 19, 20, 21, 17 and 22.

2. The antibody or fragment thereof of claim 1, wherein:
the VH comprises the amino acid sequence of SEQ ID NO:48 and the VL comprises the amino acid sequence of SEQ ID NO:52.

3. The antibody or fragment thereof of claim 2, wherein antibody or fragment thereof is a bivalent Fab antibody, or a fragment selected from the group consisting of F(ab')2, F(ab)2, Fab', Fab, Fv, and scFv.

4. The antibody or fragment thereof of any one of claims 1-3, which is humanized.

5. One or more polynucleotide(s) encoding the antibody or fragment thereof of any one of claims 1-4.

6. A cell comprising the polynucleotide(s) of claim 5.

7. An antibody-drug conjugate, comprising:
(a) an antibody or fragment thereof of any one of claims 1-4; and
(b) a conjugation moiety conjugated to the antibody or fragment thereof, wherein the conjugation moiety is selected from: a detectable marker, a drug, a toxin, a cytokine, a radionuclide, an enzyme, or a combination thereof.

8. A composition comprising the antibody or fragment thereof of any one of claims 1-4, or the antibody-drug conjugate of claim 7, and a pharmaceutically acceptable carrier.

9. The antibody or fragment thereof of any one of claims 1-4or the antibody-drug conjugate of claim 7 for treating cancer.
